# EUROPÄISCHE PATENTSCHRIFT

(11) **EP 2 874 993 B1**
(45) Veröffentlichungstag und Bekanntmachung des Hinweises auf die Patenterteilung: **24.08.2016**
(21) Anmeldenummer: 13737256.1
(22) Anmeldetag: 16.07.2013
(51) Int. Cl.: C07C 229/50, C07C 255/54, A61K 45/06, A61K 31/197, A61K 31/277, A61K 31/4015, A61K 31/415, A61K 31/421, A61K 31/45, C07D 231/12, C07D 263/22, C07D 207/27, C07D 211/76

(54) **SUBSTITUIERTE AMINOINDAN- UND AMINOTETRALIN-CARBONSÄUREN UND IHRE VERWENDUNG**
SUBSTITUTED AMINOINDANE- AND AMINOTETRALINECARBOXYLIC ACIDS AND USE THEREOF
ACIDES AMINOINDANE- ET AMINOTÉTRALINECARBOXYLIQUES SUBSTITUÉS ET LEUR UTILISATION

(30) Priorität: 20.07.2012 EP 12177283
(43) Veröffentlichungstag der Anmeldung: 27.05.2015
(73) Patentinhaber: Bayer Pharma Aktiengesellschaft, 13353 Berlin (DE)
(72) Erfinder: HÜBSCH, Walter, 42113 Wuppertal (DE); HAHN, Michael, 40764 Langenfeld (DE); VAKALOPOULOS, Alexandros, 40721 Hilden (DE); LI, Volkhart Min-Jian, 42553 Velbert (DE); WUNDER, Frank, 42117 Wuppertal (DE); STASCH, Johannes-Peter, 42651 Solingen (DE); SCHLEMMER, Karl-Heinz, 42113 Wuppertal (DE); STOLL, Friederike, 40215 Düsseldorf (DE); LINDNER, Niels, 42115 Wuppertal (DE); BECKER, Eva-Maria, 42327 Wuppertal (DE)
(74) Vertreter: BIP Patents
(86) Internationale Anmeldenummer: PCT/EP2013/065020
(87) Internationale Veröffentlichungsnummer: WO 2014/012935

(56) Entgegenhaltungen:
- WO-A1-00/35882
- WO-A1-02/070459
- WO-A1-2011/141409
- WO-A2-01/19780

## Beschreibung

Die vorliegende Anmeldung betrifft neue, substituierte Aminoindan- und Aminotetralin-Carbonsäuren, Verfahren zu ihrer Herstellung, ihre Verwendung zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention kardiovaskulärer und kardiopulmonaler Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise [O.V. Evgenov et al., Nature Rev. Drug Disc. 5 (2006), 755].

In den letzten Jahren wurden Substanzen identifiziert, die die lösliche Guanylatcyclase direkt, d.h. ohne vorherige Freisetzung von NO stimulieren. Mit dem Indazolderivat YC-1 wurde erstmals ein NO-unabhängiger, jedoch Häm-abhängiger Stimulator der sGC beschrieben [Evgenov et al., *ibid*.]. Ausgehend von YC-1 wurden weitere Substanzen gefunden, welche eine höhere Potenz als YC-1 besitzen und keine relevante Hemmung von Phosphodiesterasen (PDE) aufweisen. Dies führte zur Identifizierung der Pyrazolopyridin-Derivate BAY 41-2272, BAY 41-8543 und BAY 63-2521. Diese Verbindungen bilden gemeinsam mit den kürzlich publizierten, strukturell diversen Substanzen CMF-1571 und A-350619 die neue Klasse der sGC-Stimulatoren [Evgenov et al., *ibid*.]. Gemeinsames Charakteristikum dieser Substanzklasse ist eine NO-unabhängige und selektive Aktivierung der hämhaltigen sGC. Darüber hinaus zeigen die sGC-Stimulatoren in Kombination mit NO einen synergistischen Effekt auf die sGC-Aktivierung, welcher auf einer Stabilisierung des Nitrosyl-Häm-Komplexes basiert. Die genaue Bindungsstelle der sGC-Stimulatoren an der sGC ist bis heute Gegenstand der Diskussion. Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten Stimulatoren stimulierbar [Evgenov et al., *ibid*.].

Darüber hinaus wurden NO- und Häm-unabhängige sGC-Aktivatoren, mit BAY 58-2667 als Prototyp dieser Klasse, identifiziert. Gemeinsame Charakteristika dieser Substanzen sind, dass sie in Kombination mit NO nur einen additiven Effekt auf die Enzymaktivierung ausüben, und dass die Aktivierung des oxidierten oder hämfreien Enzyms im Vergleich zum hämhaltigen Enzym deutlich stärker ist [Evgenov et al., *ibid*.; J.P. Stasch et al., Br. J. Pharmacol. 136 (2002), 773; J.P. Stasch et al., J. Clin. Invest. 116 (2006), 2552]. Spektroskopische Untersuchungen lassen erkennen, dass BAY 58-2667 die oxidierte Hämgruppe verdrängt, die durch Schwächung der Eisen-Histidin-Bindung nur schwach an der sGC gebunden ist. Auch wurde gezeigt, dass das charakteristische sGC-Hämbindungsmotiv Tyr-x-Ser-x-Arg sowohl für die Interaktion der negativ geladenen Propionsäuren der Hämgruppe als auch für die Wirkung von BAY 58-2667 zwingend erforderlich ist. Vor diesem Hintergrund wird angenommen, dass die Bindungsstelle von BAY 58-2667 an der sGC identisch zur Bindungsstelle der Hämgruppe ist [J.P. Stasch et al., J. Clin. Invest. 116 (2006), 2552].

Die in der vorliegenden Erfindung beschriebenen Verbindungen sind nun ebenfalls in der Lage, die Häm-freie Form der löslichen Guanylatcyclase zu aktivieren. Dies wird auch dadurch belegt, dass diese neuartigen Aktivatoren einerseits am Häm-haltigen Enzym keine synergistische Wirkung mit NO zeigen und andererseits sich ihre Wirkung nicht durch den Häm-abhängigen Inhibitor der löslichen Guanylatcyclase, 1*H-*1,2,4-Oxadiazolo[4,3-a]chinoxalin-1-on (ODQ), blockieren lässt, sondern durch diesen sogar potenziert wird [vgl. O.V. Evgenov et al., Nature Rev. Drug Disc. 5 (2006), 755; J.P. Stasch et al., J. Clin. Invest. 116 (2006), 2552].

Aufgabe der vorliegenden Erfindung war somit die Bereitstellung neuer Verbindungen, welche in der oben beschriebenen Weise als Aktivatoren der löslichen Guanylatcyclase wirken und als solche insbesondere zur Behandlung und zur Prävention kardiovaskulärer und kardiopulmonaler Erkrankungen eingesetzt werden können.

Aus den Patentanmeldungen WO 01/19780-A2, WO 02/070459-A1, WO 02/070460-A1, WO 02/070461-A1, WO 02/070462-A1 und WO 02/070510-A2 sind verschiedene Aminodicarbonsäure-Derivate zur Behandlung von Herz-Kreislauf-Erkrankungen bekannt. In WO 95/ 18617-A1 und WO 00/35882-A1 werden 1-Aminoindan- und 1-Aminotetralin-Derivate zur Behandlung neurologischer Erkrankungen beschrieben. In WO 2006/104826-A2 werden N-acylierte 1-Aminoindan-5-carboxamide und 1-Aminotetralin-6-carboxamide als Glukagon-Rezeptor-Antagonisten zur Behandlung von Diabetes offenbart. In WO 2011/141409-A2 werden 8-Alkoxy-2-aminotetralin-Derivate zur Behandlung und/oder Prävention kardiovaskulärer Erkrankungen beschrieben.

Gegenstand der vorliegenden Erfindung sind Verbindungen der allgemeinen Formel (I) 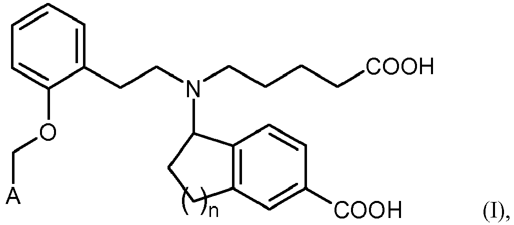 in welcher
- n: für die Zahl 1 oder 2 steht,
und
- A: für eine Gruppe der Formel 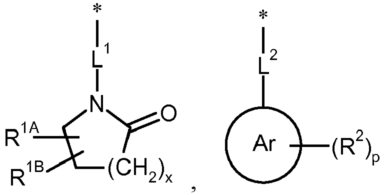
oder 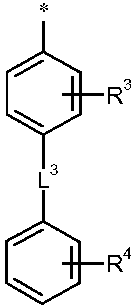 steht, worin
- *: die jeweilige Verknüpfungsstelle mit dem Rest des Moleküls kennzeichnet,
- L¹: geradkettiges (C₁-C₅)-Alkandiyl bedeutet,
- x: die Zahl 1, 2 oder 3 bedeutet, wobei eine dieser CH₂-Gruppen gegen -O- ausgetauscht sein kann,
- R^{1A} und R^{1B}: unabhängig voneinander Wasserstoff oder Methyl bedeuten,
- L²: eine Bindung oder geradkettiges (C₁-C₅)-Alkandiyl bedeutet,
- Ar: Phenyl oder 5- oder 6-gliedriges Heteroaryl mit bis zu drei Heteroatomen aus der Reihe N, O und/oder S bedeutet,
- R²: einen Substituenten ausgewählt aus der Reihe Fluor, Chlor, Brom, Cyano, (C₁-C₄)-Alkyl, Trifluormethyl, (C₁-C₄)-Alkoxy und Trifluormethoxy bedeutet,
- p: die Zahl 0, 1 oder 2 bedeutet, wobei im Falle, dass der Substituent R² zweifach auftritt, seine jeweiligen Bedeutungen gleich oder verschieden sein können,
- L³: eine Bindung, -O-, -CH₂-, -CH₂-CH₂- oder -CH=CH- bedeutet,
und
- R³ und R⁴: unabhängig voneinander Wasserstoff oder einen Substituenten ausgewählt aus der Reihe Fluor, Chlor, Brom, Cyano, (C₁-C₄)-Alkyl, Trifluormethyl, (C₁-C₄)-Alkoxy und Trifluormethoxy bedeuten,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen Säureadditionssalze üblicher Mineralsäuren, Carbonsäuren und Sulfonsäuren, wie z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, *N,N-*Diisopropylethylamin, Monoethanolamin, Diethanolamin, Triethanolamin, Dimethylaminoethanol, Diethylaminoethanol, Procain, Dicyclohexylamin, Dibenzylamin, *N*-Methylpiperidin, *N*-Methylmorpholin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²¹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Außerdem beschreibt diese Entgegenhaltung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper auf beispielsweise metabolischem oder hydrolytischem Wege zu erfindungsgemäßen Verbindungen umgesetzt werden.

Insbesondere umfasst die vorliegende Erfindung als Prodrugs hydrolysierbare Ester-Derivate der erfindungsgemäßen Carbonsäuren der Formel (I). Hierunter werden Ester verstanden, die in physiologischen Medien, unter den Bedingungen der im weiteren beschriebenen biologischen Tests und insbesondere *in vivo* auf enzymatischem oder chemischem Wege zu den freien Carbonsäuren, als den biologisch hauptsächlich aktiven Verbindungen, hydrolysiert werden können. Als solche Ester werden (C₁-C₄)-Alkylester, in welchen die Alkylgruppe geradkettig oder verzweigt sein kann, bevorzugt. Besonders bevorzugt sind Methyl-, Ethyl- oder *tert*.-Butylester.

Im Rahmen der vorliegenden Erfindung haben die Substituenten, soweit nicht anders spezifiziert, die folgende Bedeutung:
(C₁-C₄)-Alkyl steht im Rahmen der Erfindung für einen geradkettigen oder verzweigten Alkylrest mit 1 bis 4 Kohlenstoffatomen. Beispielhaft und vorzugsweise seien genannt: Methyl, Ethyl, *n*-Propyl, Isopropyl, *n*-Butyl, Isobutyl, *sec*.-Butyl und *tert.*-Butyl*.*
(C₁-C₅)-Alkandiyl, (C₁-C₄)-Alkandiyl und (C₂-C₄)-Alkandiyl stehen im Rahmen der Erfindung für einen geradkettigen, α,ω-divalenten Alkylrest mit 1 bis 5, 1 bis 4 bzw. 2 bis 4 Kohlenstoffatomen. Beispielhaft und vorzugsweise seien genannt: Methylen, Ethan-1,2-diyl (1,2-Ethylen), Propan-1,3-diyl (1,3-Propylen), Butan-1,4-diyl (1,4-Butylen) und Pentan-1,5-diyl (1,5-Pentylen).
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, n-Propoxy, Isopropoxy, *n*-Butoxy, Isobutoxy, sec.-Butoxy und *tert*.-Butoxy.
5- oder 6-gliedriges Heteroaryl steht im Rahmen der Erfindung für einen aromatischen Heterocyclus (Heteroaromaten) mit insgesamt 5 bzw. 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, 1,2-Oxazolyl (Isoxazolyl), 1,3-Oxazolyl, 1,2-Thiazolyl (Isothiazolyl), 1,3-Thiazolyl, 1,2,3-Triazolyl, 1,2,4-Triazolyl, 1,2,4-Oxadiazolyl, 1,3,4-Oxadiazolyl, 1,2,4-Thiadiazolyl, 1,3,4-Thiadiazolyl, Pyridyl, Pyrimidinyl, Pyridazinyl, Pyrazinyl, 1,2,4-Triazinyl und 1,3,5-Triazinyl.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt. Besonders bevorzugt ist die Substitution mit einem Substituenten.

Eine besondere Ausführungsform der vorliegenden Erfindung umfasst Verbindungen der Formel (I), in welcher
- n: für die Zahl 2 steht
und
- A: die oben angegebenen Bedeutungen hat,
sowie ihre Salze, Solvate und Solvate der Salze.

Eine weitere besondere Ausführungsform der vorliegenden Erfindung umfasst Verbindungen der Formel (I), in welcher
- n: für die Zahl 1 oder 2 steht,
und
- A: für eine Gruppe der Formel 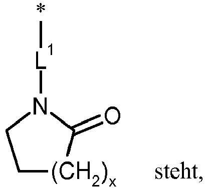
worin
- *: die Verknüpfungsstelle mit dem Rest des Moleküls kennzeichnet,
- L¹: geradkettiges (C₁-C₄)-Alkandiyl bedeutet,
und
- x: die Zahl 1 oder 2 bedeutet, wobei eine dieser CH₂-Gruppen gegen -O- ausgetauscht sein kann,
sowie ihre Salze, Solvate und Solvate der Salze.

Eine weitere besondere Ausführungsform der vorliegenden Erfindung umfasst Verbindungen der Formel (I), in welcher
- n: für die Zahl 1 oder 2 steht,
und
- A: für eine Gruppe der Formel 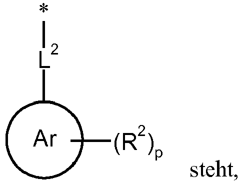
worin
- *: die Verknüpfungsstelle mit dem Rest des Moleküls kennzeichnet,
- L²: eine Bindung oder geradkettiges (C₁-C₄)-Alkandiyl bedeutet,
- Ar: Phenyl bedeutet,
- R²: einen Substituenten ausgewählt aus der Reihe Fluor, Chlor, Cyano, (C₁-C₄)-Alkyl und Trifluormethyl bedeutet,
und
- p: die Zahl 0, 1 oder 2 bedeutet,
wobei im Falle, dass der Substituent R² zweifach auftritt, seine jeweiligen Bedeutungen gleich oder verschieden sein können,
sowie ihre Salze, Solvate und Solvate der Salze.

Eine weitere besondere Ausführungsform der vorliegenden Erfindung umfasst Verbindungen der Formel (I), in welcher
- n: für die Zahl 1 oder 2 steht,
und
- A: für eine Gruppe der Formel 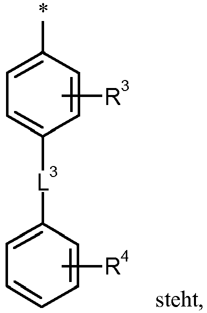
worin
- *: die Verknüpfungsstelle mit dem Rest des Moleküls kennzeichnet,
- L³: eine Bindung, -CH₂-CH₂- oder -CH=CH- bedeutet,
und
- R³ und R⁴: unabhängig voneinander Wasserstoff oder einen Substituenten ausgewählt aus der Reihe Fluor, Chlor, Cyano, (C₁-C₄)-Alkyl und Trifluormethyl bedeuten,
sowie ihre Salze, Solvate und Solvate der Salze.

Bevorzugt im Rahmen der vorliegenden Erfindung sind Verbindungen der Formel (I), in welcher
- n: für die Zahl 1 oder 2 steht,
und
- A: für eine Gruppe der Formel 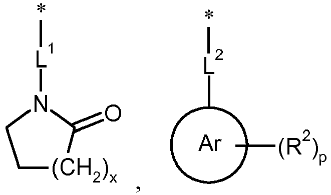
oder 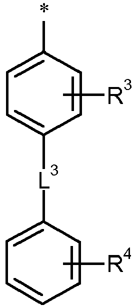 steht, worin
- *: die jeweilige Verknüpfungsstelle mit dem Rest des Moleküls kennzeichnet,
- L¹: geradkettiges (C₂-C₄)-Alkandiyl bedeutet,
- x: die Zahl 1 oder 2 bedeutet, wobei eine dieser CH₂-Gruppen gegen -O- ausgetauscht sein kann,
- L²: eine Bindung oder geradkettiges (C₁-C₄)-Alkandiyl bedeutet,
- Ar: Phenyl bedeutet,
- R²: einen Substituenten ausgewählt aus der Reihe Fluor, Chlor, Cyano, (C₁-C₄)-Alkyl und Trifluormethyl bedeutet,
- p: die Zahl 0 oder 1 bedeutet,
- L³: eine Bindung oder -CH₂-CH₂- bedeutet,
und
- R³ und R⁴: unabhängig voneinander Wasserstoff oder einen Substituenten ausgewählt aus der Reihe Fluor, Chlor, Cyano, (C₁-C₄)-Alkyl und Trifluormethyl bedeuten,
sowie ihre Salze, Solvate und Solvate der Salze.

Besonders bevorzugt im Rahmen der vorliegenden Erfindung sind Verbindungen der Formel (I), in welcher
- n: für die Zahl 1 oder 2 steht,
und
- A: für eine Gruppe der Formel 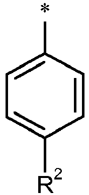
oder 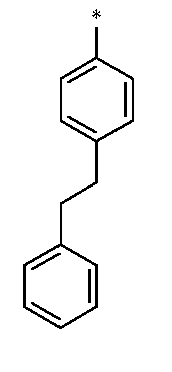 steht, worin
* die jeweilige Verknüpfungsstelle mit dem Rest des Moleküls kennzeichnet
   und
   - R²: Methyl, Ethyl, Isopropyl oder *tert*.-Butyl bedeutet,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man eine Verbindung der Formel (II) 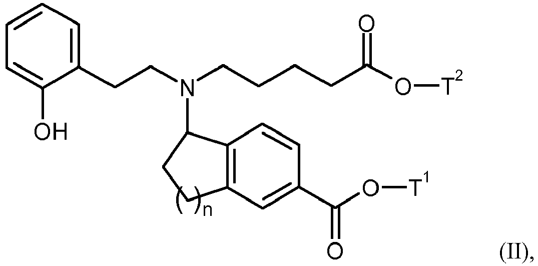 in welcher n die oben angegebenen Bedeutungen hat
und
T¹ und T² gleich oder verschieden sind und für (C₁-C₄)-Alkyl stehen,
in Gegenwart einer Base mit einer Verbindung der Formel (III) 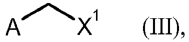 in welcher A die oben angegebenen Bedeutungen hat
und
- X¹: für eine Abgangsgruppe wie beispielsweise Chlor, Brom, Iod, Mesylat, Triflat oder Tosylat steht,
zu einer Verbindung der Formel (IV) 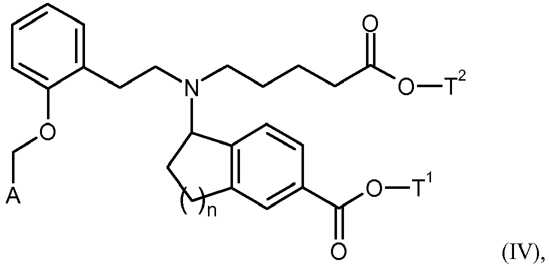 in welcher n, A, T¹ und T² jeweils die oben angegebenen Bedeutungen haben,
umsetzt und diese dann durch Hydrolyse der Ester-Gruppierungen -C(O)OT¹ und -C(O)OT² in die entsprechende Dicarbonsäure der Formel (I) überführt
und die so erhaltenen Verbindungen der Formel (I) gegebenenfalls in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

Als inerte Lösungsmittel für den Verfahrensschritt (II) + (III) → (IV) eignen sich beispielsweise Ether wie Diethylether, Diisopropylether, Methyl-*tert*.-butylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Heptan, Cyclohexan oder Erdölfraktionen, oder dipolar-aprotische Lösungsmittel wie Aceton, Methylethylketon, Acetonitril, *N,N-*Dimethylformamid (DMF), *N,N-*Dimethylacetamid (DMA), Dimethylsulfoxid (DMSO), *N*,*N*'-Dimethylpropylenharnstoff (DMPU) oder *N*-Methyl-pyrrolidinon (NMP). Ebenso ist es möglich, Gemische solcher Lösungsmittel einzusetzen. Bevorzugt wird Acetonitril oder Dimethylformamid verwendet.

Für den Verfahrensschritt (II) + (III) → (IV) geeignete Basen sind insbesondere Alkalicarbonate wie Natrium-, Kalium- oder Cäsiumcarbonat, Alkali-Alkoholate wie Natrium- oder Kaliummethanolat, Natrium- oder Kaliumethanolat oder Natrium- oder Kalium-*tert*.-butylat, Alkalihydride wie Natrium- oder Kaliumhydrid, Amide wie Natriumamid, Lithium- oder Kalium-bis(trimethylsilyl)-amid oder Lithiumdiisopropylamid, oder metallorganische Verbindungen wie *n*-Butyllithium oder Phenyllithium. Bevorzugt wird Natrium-, Kalium- oder Cäsiumcarbonat als Base eingesetzt. Gegebenenfalls ist der Zusatz eines Alkylierungskatalysators, wie beispielsweise Lithiumbromid, Natrium- oder Kaliumiodid, Tetra-*n*-butylammoniumbromid oder Benzyltriethylammoniumchlorid, von Vorteil.

Die Umsetzung (II) + (III) → (IV) wird im Allgemeinen in einem Temperaturbereich von 0°C bis +150°C, bevorzugt bei +20°C bis +100°C durchgeführt.

Die Hydrolyse der Ester-Gruppen -C(O)OT¹ und -C(O)OT² im Verfahrensschritt (IV) → (I) wird nach üblichen Methoden durchgeführt, indem man die Ester in inerten Lösungsmitteln mit Säuren oder Basen behandelt, wobei bei letzterer Variante die zunächst entstehenden Salze durch Behandeln mit Säure in die freien Carbonsäuren überführt werden. Im Falle der *tert*.-Butylester erfolgt die Esterspaltung bevorzugt mit Säuren.

Bei unterschiedlichen Gruppen T¹ und T² kann die Hydrolyse gegebenenfalls simultan in einer Eintopf-Reaktion oder in zwei separaten Reaktionsschritten durchgeführt werden.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, *n*-Butanol oder *tert*.-Butanol, oder Ether wie Diethylether, Tetrahydrofuran, 1,4-Dioxan oder 1,2-Dimethoxyethan, oder andere Lösungsmittel wie Dichlormethan, Aceton, Methylethylketon, *N,N-*Dimethylformamid oder Dimethylsulfoxid. Ebenso ist es möglich, Gemische dieser Lösungsmittel einzusetzen. Im Falle einer basischen Ester-Hydrolyse werden bevorzugt Gemische von Wasser mit Dioxan, Tetrahydrofuran, Methanol, Ethanol, Dimethylformamid und/oder Dimethylsulfoxid verwendet. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser eingesetzt.

Als Basen sind die üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt sind Lithium-, Natrium- oder Kaliumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von -20°C bis +120°C, bevorzugt bei 0°C bis +80°C.

Die zuvor beschriebenen Verfahrensschritte können bei normalem, bei erhöhtem oder bei erniedrigtem Druck durchgeführt werden (z.B. im Bereich von 0.5 bis 5 bar); im Allgemeinen arbeitet man jeweils bei Normaldruck.

Die Verbindungen der Formel (II) ihrerseits können dadurch hergestellt werden, dass man eine Keto-Verbindung der Formel (V) 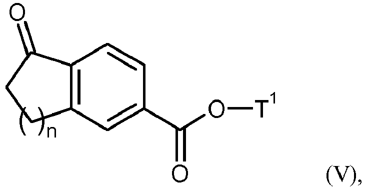 in welcher n und T¹ die oben angegebenen Bedeutungen haben,
im Zuge einer reduktiven Aminierung mit 2-(2-Methoxyphenyl)ethylamin (VI) 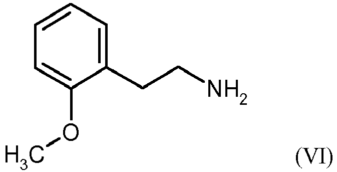 zu einem sekundären Amin der Formel (VII) 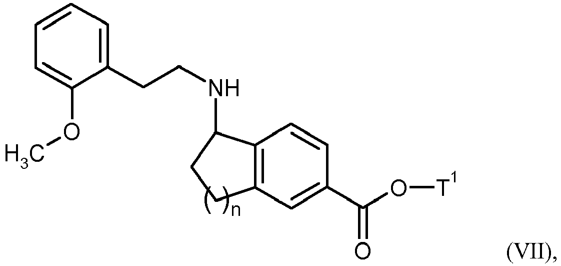 in welcher n und T¹ die oben angegebenen Bedeutungen haben,
umsetzt, nachfolgend in Gegenwart einer Base mit einem 5-Halovaleriansäureester der Formel (VIII) 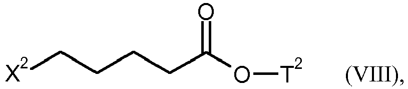 in welcher T² die oben angegebene Bedeutung hat
und
- X²: für Chlor, Brom oder Iod steht,
zu einem tertiären Amin der Formel (IX) 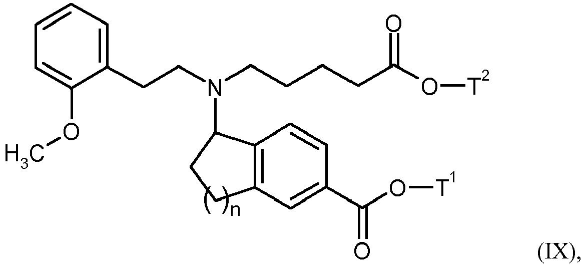 in welcher n, T¹ und T² jeweils die oben angegebenen Bedeutungen haben,
alkyliert und anschließend die phenolische Methylether-Gruppierung durch Behandlung mit Bortribromid oder Bromwasserstoff abspaltet.

Die Umsetzung (V) + (VI) → (VII) erfolgt in einem für reduktive Aminierungen üblichen, unter den Reaktionsbedingungen inerten Lösungsmittel, gegebenenfalls in Gegenwart einer Säure und/ oder eines wasserentziehenden Mittels als Katalysator. Zu diesen Lösungsmitteln gehören beispielsweise Tetrahydrofuran, Toluol, Dichlormethan, 1,2-Dichlorethan, *N,N*-Dimethylformamid und Alkohole wie Methanol, Ethanol, *n*-Propanol oder Isopropanol; auch ist es möglich, Gemische solcher Lösungsmittel einzusetzen. Bevorzugt werden Methanol oder Ethanol verwendet. Als Katalysator kommen gebräuchliche organische Säuren wie Essigsäure oder *p*-Toluolsulfonsäure in Betracht.

Als Reduktionsmittel für diese Aminierungsreaktion eignen sich insbesondere Borhydride wie beispielsweise Natriumborhydrid, Natriumcyanoborhydrid, Natriumtriacetoxyborhydrid oder Tetra-*n*-butylammoniumborhydrid; bevorzugt wird Natriumborhydrid eingesetzt.

Die Umsetzung (V) + (VI) → (VII) erfolgt im Allgemeinen in einem Temperaturbereich von -20°C bis +50°C, bevorzugt bei 0°C bis +30°C.

Die Alkylierung im Verfahrensschritt (VII) + (VIII) → (IX) wird unter analogen Reaktionsbedingungen bezüglich Lösungsmittel, Base und Temperatur durchgeführt, wie zuvor bei der Umsetzung (II) + (III) → (IV) beschrieben. Bevorzugt werden hier als Base Alkalicarbonate und als Lösungsmittel Acetonitril eingesetzt. Die Alkylierung erfolgt in der Regel in einem Temperaturbereich von +50°C bis +100°C.

Die Spaltung der phenolischen Methylether-Gruppe im Verfahrensschritt (IX) → (II) erfolgt nach üblichen Methoden durch Behandlung mit Bortribromid in Dichlormethan bei -20°C bis +10°C oder durch Erhitzen mit einer Lösung von Bromwasserstoff in Eisessig oder Wasser auf +100°C bis +130°C. Sollten unter diesen Reaktionsbedingungen gleichzeitig auch die Ester-Gruppierungen -C(O)OT¹ und -C(O)OT² ganz oder teilweise zu den entsprechenden freien Carbonsäuren der Formel (X) 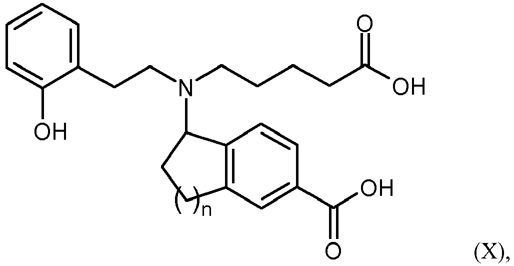 in welcher n die oben angegebene Bedeutung hat,
gespalten werden, so können diese beispielsweise durch nachfolgende Behandlung mit Methanol oder Ethanol in Gegenwart von Chlorwasserstoff oder Thionylchlorid wieder rück-verestert werden.

Die zuvor beschriebenen Umsetzungen können bei normalem, bei erhöhtem oder bei erniedrigtem Druck durchgeführt werden (z.B. im Bereich von 0.5 bis 5 bar); im Allgemeinen arbeitet man jeweils bei Normaldruck.

Eine Trennung der erfindungsgemäßen Verbindungen in die entsprechenden Enantiomere und/ oder Diastereomere kann gegebenenfalls, je nach Zweckmäßigkeit, auch bereits auf der Stufe der Verbindungen (II), (IV), (VII), (IX) oder (X) erfolgen, welche dann in separierter Form entsprechend den zuvor beschriebenen Verfahrenssequenzen weiter umgesetzt werden. Eine solche Auftrennung der Stereoisomeren läßt sich nach üblichen, dem Fachmann bekannten Methoden durchführen. Vorzugsweise werden chromatographische Verfahren an achiralen bzw. chiralen Trennphasen angewandt; im Falle von Carbonsäuren als Zwischen- oder Endprodukten kann alternativ auch eine Trennung über diastereomere Salze mit Hilfe chiraler Basen erfolgen.

Die Verbindungen der Formel (V) sind nach Literaturvorschriften zugänglich [siehe z.B. für 1-Oxo-indan-5-carbonsäureethylester (*n* = *1*): R. Takeuchi und H. Yasue, J. Org. Chem. 1993, 58 (20), 5386-5392; für 5-Oxo-5,6,7,8-tetrahydronaphthalin-2-carbonsäuremethylester (*n* = *2*): U. Gerlach und T. Wollmann, Tetrahedron Lett. 1992, 33 (38), 5499-5502].

Die Verbindungen der Formeln (III), (VI) und (VIII) sind entweder kommerziell erhältlich oder als solche in der Literatur beschrieben, oder sie können auf für den Fachmann offenkundigem Wege in Analogie zu in der Literatur publizierten Methoden hergestellt werden. Zahlreiche detaillierte Vorschriften befinden sich auch im Experimentellen Teil im Abschnitt zur Herstellung der Ausgangsverbindungen und Intermediate.

Die Herstellung der erfindungsgemäßen Verbindungen kann durch das folgende Reaktionsschema beispielhaft veranschaulicht werden:

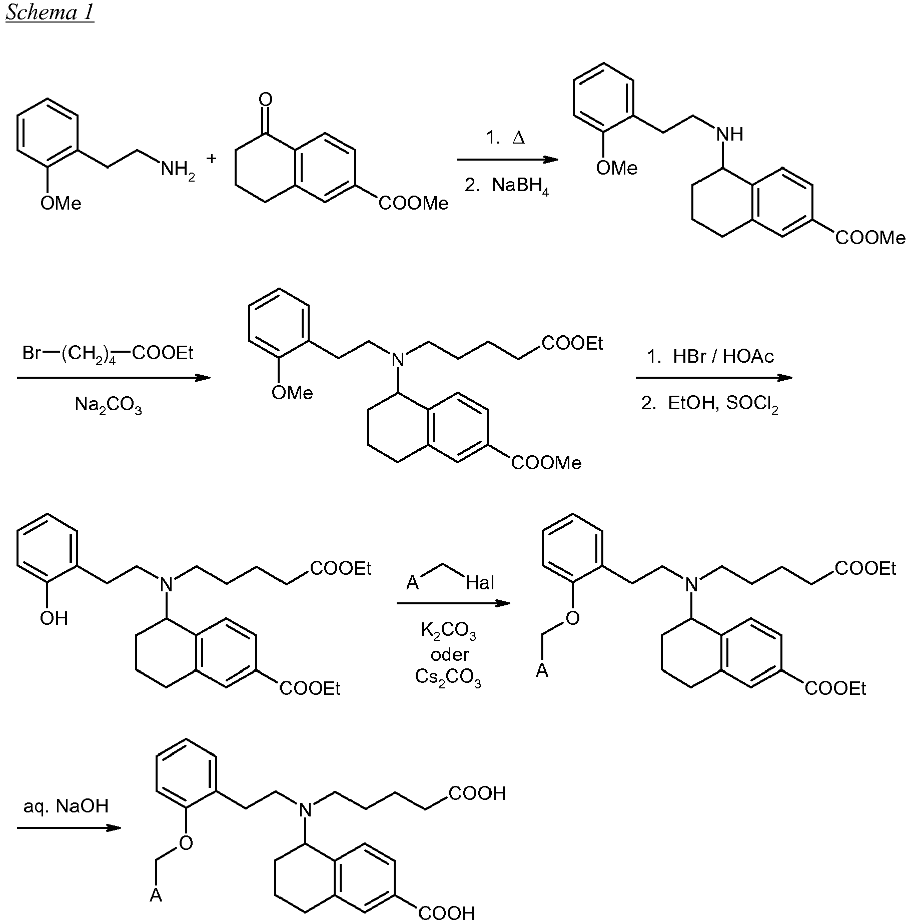

Die erfindungsgemäßen Verbindungen besitzen wertvolle pharmakologische Eigenschaften und können zur Vorbeugung und Behandlung von Erkrankungen bei Menschen und Tieren verwendet werden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Die erfindungsgemäßen Verbindungen stellen potente Aktivatoren der löslichen Guanylatcyclase dar. Sie führen zu einer Gefäßrelaxation, zu einer Thrombozytenaggregationshemmung und zu einer Blutdrucksenkung sowie zu einer Steigerung des koronaren Blutflusses und der Mikrozirkulation. Diese Wirkungen sind über eine direkte, Häm-unabhängige Aktivierung der löslichen Guanylatcyclase und einen intrazellulären cGMP-Anstieg vermittelt.

Die erfindungsgemäßen Verbindungen eignen sich in besonderem Maße zur Behandlung und/oder Prävention von kardiovaskulären, pulmonalen, thromboembolischen und fibrotischen Erkrankungen.

Die erfindungsgemäßen Verbindungen können daher in Arzneimitteln eingesetzt werden zur Behandlung und/oder Prävention von kardiovaskulären Erkrankungen, wie beispielsweise Bluthochdruck (Hypertonie), Herzinsuffizienz, koronare Herzerkrankung, stabile und instabile Angina pectoris, pulmonale arterielle Hypertonie (PAH) und andere Formen der pulmonalen Hypertonie (PH), renale Hypertonie, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden des Grades I-III, supraventrikuläre Tachyarrhythmie, Vorhofflimmern, Vorhofflattern, Kammerflimmern, Kammerflattern, ventrikuläre Tachyarrhythmie, Torsade de pointes-Tachykardie, Extrasystolen des Vorhofs und des Ventrikels, AV-junktionale Extrasystolen, Sick-Sinus-Syndrom, Synkopen, AV-Knoten-Reentry-Tachykardie, Wolff-Parkinson-White-Syndrom, akutes Koronarsyndrom (ACS), autoimmune Herzerkrankungen (Perikarditis, Endokarditis, Valvolitis, Aortitis, Kardiomyopathien), Boxerkardiomyopathie, Aneurysmen, Schock wie kardiogener Schock, septischer Schock und anaphylaktischer Schock, ferner zur Behandlung und/oder Prävention von thromboembolischen Erkrankungen und Ischämien, wie myokardiale Ischämie, Myokardinfarkt, Hirnschlag, Herzhypertrophie, transistorische und ischämische Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, periphere Durchblutungsstörungen, Reperfusionsschäden, arterielle und venöse Thrombosen, Mikroalbuminurie, Herzmuskelschwäche, endotheliale Dysfunktion, mikro- und makrovaskuläre Schädigungen (Vaskulitis), sowie zur Verhinderung von Restenosen beispielsweise nach Thrombolyse-Therapien, percutan-transluminalen Angioplastien (PTA), percutan-transluminalen Koronarangioplastien (PTCA), Herztransplantationen und Bypass-Operationen.

Im Sinne der vorliegenden Erfindung umfasst der Begriff Herzinsuffizienz sowohl akute als auch chronische Erscheinungsformen der Herzinsuffizienz wie auch spezifische oder verwandte Krankheitsformen hiervon, wie akute dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Darüber hinaus können die erfindungsgemäßen Verbindungen auch zur Behandlung und/oder Prävention von Arteriosklerose, Lipidstoffwechselstörungen, Hypolipoproteinämien, Dyslipidämien, Hypertriglyceridämien, Hyperlipidämien, kombinierten Hyperlipidämien, Hypercholesterolämien, Abetalipoproteinämie, Sitosterolämie, Xanthomatose, Tangier-Krankheit, Fettsucht (Adipositas), Fettleibigkeit (Obesitas) sowie des Metabolischen Syndroms eingesetzt werden.

Weiterhin können die erfindungsgemäßen Verbindungen zur Behandlung und/oder Prävention von primärem und sekundärem Raynaud-Phänomen, Mikrozirkulationsstörungen, Claudicatio, Tinnitus, peripheren und autonomen Neuropathien, diabetischen Mikroangiopathien, diabetischer Retinopathie, diabetischen Geschwüren an den Extremitäten, Gangrän, CREST-Syndrom, Erythematose, Onychomykose sowie von rheumatischen Erkrankungen verwendet werden.

Die erfindungsgemäßen Verbindungen können darüber hinaus zur Verhinderung von ischämie- und/oder reperfusionsbedingten Schädigungen von Organen oder Geweben sowie als Zusatzstoffe für Perfusions- und Konservierungslösungen von Organen, Organteilen, Geweben oder Gewebeteilen menschlichen oder tierischen Ursprungs, insbesondere bei chirurgischen Eingriffen oder im Bereich der Transplantationsmedizin, Verwendung finden.

Die erfindungsgemäßen Verbindungen eignen sich außerdem zur Behandlung und/oder Prävention von Nierenerkrankungen, insbesondere von Niereninsuffizienz und Nierenversagen. Im Sinne der vorliegenden Erfindung umfassen die Begriffe Niereninsuffizienz und Nierenversagen sowohl akute als auch chronische Erscheinungsformen hiervon wie auch diesen zugrundeliegende oder verwandte Nierenerkrankungen, wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantat-Abstoßung und Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Darüber hinaus sind die erfindungsgemäßen Verbindungen zur Behandlung und/oder Prävention von Erkrankungen des Urogenitalsystems geeignet, wie beispielsweise benignes Prostata-Syndrom (BPS), benigne Prostatahyperplasie (BPH), benigne Prostatavergrößerung (BPE), Blasenentleerungsstörungen (BOO), untere Harnwegssyndrome (LUTS), neurogene überaktive Blase (OAB), Inkontinenz wie beispielsweise Misch-, Drang-, Stress- oder Überlauf-Inkontinenz (MUI, UUI, SUI, OUI), Beckenschmerzen sowie erektile Dysfunktion und weibliche sexuelle Dysfunktion.

Die erfindungsgemäßen Verbindungen eignen sich auch zur Behandlung und/oder Prävention von asthmatischen Erkrankungen, chronisch-obstruktiven Atemwegserkrankungen (COPD), des akuten Atemwegssyndroms (ARDS) und der akuten Lungenschädigung (ALI), alpha-1-Antitrypsin-Defizienz (AATD), Lungenfibrose, Lungenemphysem (z.B. durch Zigarettenrauch induziertes Lungenemphysem) und zystischer Fibrose (CF) sowie von pulmonaler arterieller Hypertonie (PAH) und anderen Formen der pulmonalen Hypertonie (PH), einschließlich der mit Linksherzerkrankung, HIV, Sichelzellanämie, Thromboembolien, Sarkoidose, COPD oder Lungenfibrose assoziierten pulmonalen Hypertonie.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierte Lern- und Gedächtnisstörungen, altersassoziierte Gedächtnisverluste, vaskuläre Demenz, Schädel-Hirn-Trauma, Schlaganfall, Demenz, die nach Schlaganfällen auftritt ("post stroke dementia"), post-traumatisches Schädel-Hirn-Trauma, allgemeine Konzentrationsstörungen, Konzentrationsstörungen bei Kindern mit Lern- und Gedächtnisproblemen, Alzheimer'sche Krankheit, Demenz mit Lewy-Körperchen, Demenz mit Degeneration der Frontallappen einschließlich des Pick's-Syndroms, Parkinson'sche Krankheit, progressiver nuclear palsy, Demenz mit corticobasaler Degeneration, Amyolateralsklerose (ALS), Huntington'sche Krankheit, Demyelinisation, Multiple Sklerose, thalamische Degeneration, Creutzfeld-Jacob-Demenz, HIV-Demenz, Schizophrenie mit Demenz oder Korsakoff-Psychose. Sie eignen sich auch zur Behandlung und/oder Prävention von Erkrankungen des Zentralnervensystems wie Angst-, Spannungs- und Depressionszuständen, zentralnervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen anti-inflammatorische Wirkung und können daher als entzündungshemmende Mittel zur Behandlung und/oder Prävention von Sepsis (SIRS), multiplem Organversagen (MODS, MOF), entzündlichen Erkrankungen der Niere, chronischen Darmentzündungen (IBD, Morbus Crohn, Colitis ulcerosa), Pankreatitis, Peritonitis, rheumatoiden Erkrankungen, entzündlichen Hauterkrankungen und entzündlichen Augenerkrankungen eingesetzt werden.

Die erfindungsgemäßen Verbindungen sind ferner zur Behandlung und/oder Prävention von fibrotischen Erkrankungen der inneren Organe, wie beispielsweise der Lunge, des Herzens, der Niere, des Knochenmarks und insbesondere der Leber, sowie von dermatologischen Fibrosen und fibrotischen Erkrankungen des Auges geeignet. Im Sinne der vorliegenden Erfindung umfasst der Begriff fibrotische Erkrankungen insbesondere solche Erkrankungen wie Leberfibrose, Leberzirrhose, Lungenfibrose, Endomyokardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung, Naevi, diabetische Retinopathie, proliferative Vitroretinopathie und Erkrankungen des Bindegewebes (z.B. Sarkoidose). Die erfindungsgemäßen Verbindungen können ebenso verwendet werden zur Förderung der Wundheilung, zur Bekämpfung postoperativer Narbenbildung, z.B. nach Glaukom-Operationen, und zu kosmetischen Zwecken bei alternder oder verhornender Haut.

Aufgrund ihres Wirkprofils eignen sich die erfindungsgemäßen Verbindungen insbesondere zur Behandlung und/oder Prävention kardiovaskulärer Erkrankungen wie Herzinsuffizienz, Angina pectoris, Hypertonie und pulmonale Hypertonie, sowie von thromboembolischen Erkrankungen und Ischämien, Gefäßerkrankungen, Mikrozirkulationsstörungen, Niereninsuffizienz, fibrotischen Erkrankungen und Arteriosklerose.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Arzneimittel, enthaltend mindestens eine der erfindungsgemäßen Verbindungen, zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer hierin beschriebene Gegenstand ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 4-Inhibitoren wie Roflumilast oder Revamilast und PDE 5-Inhibitoren wie Sildenafil, Vardenafil, Tadalafil, Udenafil, Dasantafil, Avanafil, Mirodenafil oder Lodenafil;
- NO-unabhängige, jedoch Häm-abhängige Stimulatoren der Guanylatcyclase, wie insbesondere Riociguat sowie die in WO 00/06568, WO 00/06569, WO 02/42301, WO 03/095451, WO 2011/147809, WO 2012/004258, WO 2012/028647 und WO 2012/059549 beschriebenen Verbindungen;
- Prostacyclin-Analoga und IP-Rezeptor-Agonisten, wie beispielhaft und vorzugsweise Iloprost, Beraprost, Treprostinil, Epoprostenol oder NS-304;
- Endothelin-Rezeptor-Antagonisten, wie beispielhaft und vorzugsweise Bosentan, Darusentan, Ambrisentan oder Sitaxsentan;
- Verbindungen, die die humane neutrophile Elastase (HNE) inhibieren, wie beispielhaft und vorzugsweise Sivelestat oder DX-890 (Reltran);
- die Signaltransduktionskaskade inhibierende Verbindungen, insbesondere aus der Gruppe der Tyrosinkinase-Inhibitoren, wie beispielhaft und vorzugsweise Dasatinib, Nilotinib, Bosutinib, Regorafenib, Sorafenib, Sunitinib, Cediranib, Axitinib, Telatinib, Imatinib, Brivanib, Pazopanib, Vatalanib, Gefitinib, Erlotinib, Lapatinib, Canertinib, Lestaurtinib, Pelitinib, Semaxanib, Masitinib oder Tandutinib;
- die Rho-Kinase inhibierende Verbindungen, wie beispielhaft und vorzugsweise Fasudil, Y-27632, SLx-2119, BF-66851, BF-66852, BF-66853, KI-23095 oder BA-1049;
- anti-obstruktiv wirkende Mittel, wie sie z.B. zur Therapie einer chronisch-obstruktiven Lungenerkrankung (COPD) oder eines Asthma bronchiale eingesetzt werden, wie beispielhaft und vorzugsweise inhalativ oder systemisch angewendete beta-Rezeptor-Mimetika oder inhalativ angewendete anti-muscarinerge Substanzen;
- entzündungshemmmende und/oder immunsuppressive Mittel, wie sie z.B. zur Therapie einer chronisch-obstruktiven Lungenerkrankung (COPD), eines Asthma bronchiale oder einer Lungenfibrose eingesetzt werden, wie beispielhaft und vorzugsweise systemisch oder inhalativ angewendete Corticosteroide;
- Chemotherapeutika, wie sie z.B. zur Therapie von Neubildungen (Neoplasien) der Lunge oder anderer Organe eingesetzt werden;
- Wirkstoffe, die zur systemischen und/oder inhalativen Behandlung von Lungenerkrankungen eingesetzt werden, wie beispielsweise bei zystischer Fibrose (alpha-1-Antitrypsin, Aztreonam, Ivacaftor, Lumacaftor, Ataluren, Amikacin, Levofloxacin), chronisch-obstruktiven Atemwegserkrankungen (COPD) (LAS40464, PT003, SUN-101), akutem Atemwegssyndrom (ARDS) und akuter Lungenschädigung (ALI) (Interferon-beta-1a, Traumakine), obstruktiver Schlafapnoe (VI-0521), Bronchiektasie (Mannitol, Ciprofloxacin), Bronchiolitis obliterans (Cyclosporin, Aztreonam) und Sepsis (Pagibaximab, Voluven, ART-123);
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Dosieraerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale, die intrapulmonale (inhalative) und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht. Bei intrapulmonaler Applikation beträgt die Menge im Allgemeinen etwa 0.1 bis 50 mg je Inhalation.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Ac: Acetyl
- aq.: wässrig, wässrige Lösung
- ATP: Adenosin-5'-triphosphat
- Brij^{®}: Polyethylenglycoldodecylether
- BSA: bovines Serumalbumin
- Bsp.: Beispiel
- c: Konzentration
- cat.: katalytisch
- DMF: *N,N-*Dimethylformamid
- DMSO: Dimethylsulfoxid
- d. Th.: der Theorie (bei Ausbeute)
- DTT: Dithiothreitol
- ee: Enantiomerenüberschuss
- *ent*: enantiomerenrein, Enantiomer
- eq.: Äquivalent(e)
- ESI: Elektrospray-Ionisation (bei MS)
- Et: Ethyl
- GTP: Guanosin-5'-triphosphat
- h: Stunde(n)
- Hal: Halogen
- HOAc: Essigsäure
- HPLC: Hochdruck-, Hochleistungsflüssigchromatographie
- LC-MS: Flüssigchromatographie-gekoppelte Massenspektroskopie
- Me: Methyl
- min: Minute(n)
- MS: Massenspektroskopie
- NMR: Kernresonanzspektroskopie
- quant.: quantitativ (bei Ausbeute)
- *rac*: racemisch, Racemat
- RP: reverse phase (Umkehrphase, bei HPLC)
- RT: Raumtemperatur
- Rₜ: Retentionszeit (bei HPLC)
- s.o.: siehe oben
- TEA: Triethanolamin
- TFA: Trifluoressigsäure
- THF: Tetrahydrofuran
- UV: Ultraviolett-Spektroskopie
- v/v: Volumen zu Volumen-Verhältnis (einer Lösung)
- zus.: zusammen

### HPLC- und LC-MS-Methoden:

### Methode 1 (präparative HPLC):

Säule: Grom-Sil C18 10 µm, 250 mm x 30 mm; Eluent A: Wasser + 0.1% Ameisensäure, Eluent B: Acetonitril; Programm: 0-5 min 10% B, 5-38 min Gradient bis 95% B; Fluss: 50 ml/min; UV-Detektion: 210 nm.

### Methode 2 (LC-MS):

Instrument: Waters Acquity SQD UPLC System; Säule: Waters Acquity UPLC HSS T3 1.8 µ, 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 3 (LC-MS):

Gerätetyp MS: Waters Micromass Quattro Micro; Gerätetyp HPLC: Agilent 1100 Serie; Säule: Thermo Hypersil GOLD 3 µ, 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 4 (LC-MS):

Instrument: Micromass Quattro Premier mit Waters UPLC Acquity; Säule: Thermo Hypersil GOLD 1.9 µ, 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 5 (chirale analytische HPLC):

Stationäre Phase: Daicel OD-H; Säule: 250 mm x 4 mm; UV-Detektion: 230 nm; Elutionsmittel: Isopropanol/Isohexan 30:70 (v/v); Fluss: 1.0 ml/min.

### Methode 6 (chirale analytische HPLC):

Stationäre Phase: Daicel OJ-H, 5 µm; Säule: 250 mm x 4 mm; UV-Detektion: 230 nm; Elutionsmittel: Isohexan/Methanol/Ethanol 50:25:25 (v/v/v); Fluss: 1.0 ml/min.

### Methode 7 (chirale analytische HPLC):

Stationäre Phase: Daicel Chiralpak IA; Säule: 250 mm x 4 mm; UV-Detektion: 230 nm; Elutionsmittel: Ethanol/Methyl-*tert*.-butylether 75:25 (v/v); Fluss: 1.0 ml/min.

### Methode 8 (präparative LC-MS):

Instrument MS: Waters, Instrument HPLC: Waters; Säule: Waters X-Bridge C18 5 µm, 18 mm x 50 mm; Eluent A: Wasser + 0.05% Triethylamin, Eluent B: Acetonitril + 0.05% Triethylamin; Gradient: 0.0 min 95% A → 0.15 min 95% A → 8.0 min 5% A → 9.0 min 5% A; Fluss: 40 ml/ min; UV-Detektion: DAD, 210-400 nm.

### Methode 9 (präparative LC-MS):

Instrument MS: Waters, Instrument HPLC: Waters; Säule: Phenomenex Luna 5 µ C18(2) 100A, 50 mm x 21.2 mm; Eluent A: Wasser + 0.05% Ameisensäure, Eluent B: Acetonitril + 0.05% Ameisensäure; Gradient: 0.0 min 95% A → 0.15 min 95% A → 8.0 min 5% A → 9.0 min 5% A; Fluss: 40 ml/min; UV-Detektion: DAD, 210-400 nm.

### Methode 10 (LC-MS):

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril + 0.025% Ameisensäure; Gradient: 0.0 min 98% A → 0.9 min 25% A → 1.0 min 5% A → 1.4 min 5% A → 1.41 min 98% A → 1.5 min 98% A; Ofen: 40°C; Fluss: 0.60 ml/min; UV-Detektion: DAD, 210 nm.

### Ausgangsverbindungen und Intermediate:

### Beispiel 1A

### rac-5-{[2-(2-Methoxyphenyl)ethyl]amino}-5,6,7,8-tetrahydronaphthalin-2-carbonsäuremethylester

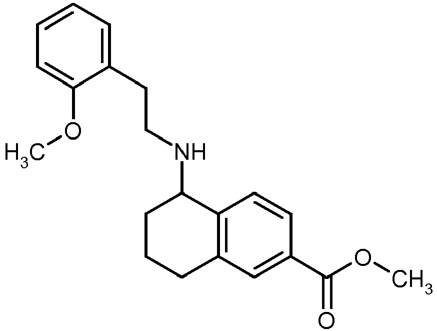

15.2 g (74.5 mmol) Methyl-5-oxo-5,6,7,8-tetrahydronaphthalin-2-carboxylat [U. Gerlach und T. Wollmann, Tetrahedron Lett. 1992, 33 (38), 5499-5502] und 11.5 g (74.5 mmol) 2-(2-Methoxyphenyl)ethylamin wurden in 50 ml Ethanol 2 h unter Rückfluss erhitzt. Danach wurde im Vakuum bis zur Trockene eingeengt, der Rückstand in 300 ml Methanol aufgenommen und die Suspension innerhalb von 20 min bei RT unter leichter externer Kühlung portionsweise mit 5.6 g (149 mmol) Natriumborhydrid versetzt. Die bräunliche Lösung wurde 24 h bei RT gerührt, dann in 900 ml Wasser gegossen und dreimal mit Ethylacetat extrahiert. Die vereinigten organischen Phasen wurden über Natriumsulfat getrocknet und eingeengt. Der Rückstand (24.5 g) wurde durch Flash-Chromatographie an Kieselgel mit Cyclohexan/Ethylacetat 2:1 als Laufmittel gereinigt.
Ausbeute: 21.0 g (83% d. Th.)
LC-MS (Methode 4): Rₜ = 0.87 min; MS (ESIpos): m/z = 340 [M+H]⁺
¹H-NMR (400 MHz, DMSO-d₆): δ [ppm] = 7.56-7.79 (m, 2H), 7.47 (d, 1H), 7.04-7.28 (m, 2H), 6.94 (d, 1H), 6.86 (t, 1H), 3.82 (s, 3H), 3.73 (br. s, 1H), 2.61-2.91 (m, 6H), 1.59-1.95 (m, 5H).

### Beispiel 2A

### rac-5-{(5-Ethoxy-5-oxopentyl)[2-(2-methoxyphenyl)ethyl]amino}-5,6,7,8-tetrahydronaphthalin-2-carbonsäuremethylester

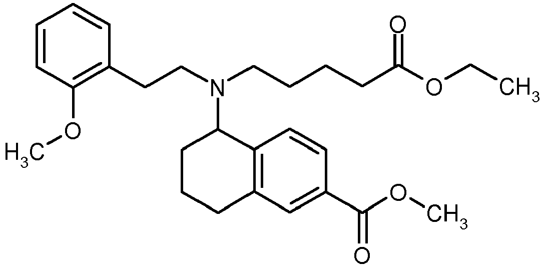

9.0 g (26.5 mmol) der Verbindung aus Beispiel 1A wurden in 100 ml Acetonitril gelöst und mit 4.6 ml (6.1 g, 29.2 mmol) 5-Bromvaleriansäureethylester sowie 5.65 g (53 mmol) Natriumcarbonat versetzt. Das Gemisch wurde 3 Tage unter Rückfluss gerührt. Nach Zugabe von weiteren 1.3 ml (8.2 mmol) 5-Bromvaleriansäureethylester und 1.6 g (15.1 mmol) Natriumcarbonat wurde nochmals über Nacht unter Rückfluss gerührt. Es wurden erneut die gleichen Mengen an 5-Bromvaleriansäureethylester und Natriumcarbonat zugegeben und wiederum eine Nacht unter Rückfluss erhitzt, bis nach LC-MS kein Edukt mehr nachzuweisen war. Danach wurde der Ansatz eingeengt, mit Wasser versetzt und mehrmals mit Ethylacetat extrahiert. Die vereinigten organischen Phasen wurden über Natriumsulfat getrocknet und eingeengt. Das Rohprodukt (14.6 g) wurde durch Flash-Chromatographie an Kieselgel mit Cyclohexan/Ethylacetat 5:1 als Laufmittel gereinigt.
Ausbeute: 11.6 g eines Öls (87% d. Th.)
LC-MS (Methode 2): Rₜ = 0.93 min; MS (ESIpos): m/z = 468 [M+H]⁺
¹H-NMR (400 MHz, DMSO-d₆): δ [ppm] = 7.55-7.70 (m, 3H), 6.95-7.21 (m, 2H), 6.74-6.93 (m, 2H), 3.90-4.14 (m, 4H), 3.82 (s, 3H), 3.64 (s, 3H), 3.54 (t, 1H), 2.57-2.82 (m, 4H), 2.33 (t, 1H), 2.13-2.26 (m, 2H), 1.87-2.07 (m, 2H), 1.74-1.87 (m, 1H), 1.34-1.71 (m, 8H), 1.06-1.21 (m, 4H) [*weitere Signale durch DMSO-Peak überlagert*]*.*

### Beispiel 3A

### rac-5-{[2-(2-Hydroxyphenyl)ethyl](5-methoxy-5-oxopentyl)amino}-5,6,7,8-tetrahydronaphthalin-2-carbonsäuremethylester

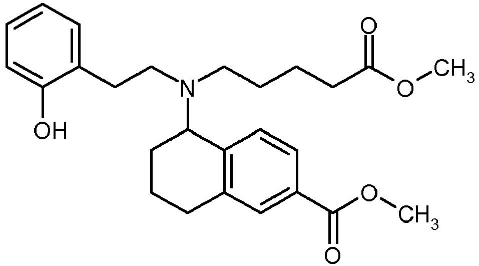

653 mg (1.4 mmol) der Verbindung aus Beispiel 2A wurden unter Argon in 12 ml Dichlormethan gelöst und auf 0°C abgekühlt. Es wurden 4.6 ml (4.6 mmol) einer 1 M Lösung von Bortribromid in Dichlormethan langsam hinzugetropft und das Gemisch 4 h bei 0°C nachgerührt (klare gelbe Lösung). Anschließend wurde mit 7 ml abs. Methanol versetzt und über Nacht unter Rückfluss erhitzt. Der Ansatz wurde dann im Vakuum bis zur Trockene eingeengt und der Rückstand zwischen Ethylacetat und 10%-iger wässriger Natriumcarbonat-Lösung verteilt. Die wässrige Phase wurde nochmals mit Ethylacetat extrahiert, und die vereinigten organischen Phasen wurden mit gesättigter Kochsalz-Lösung gewaschen und über Natriumsulfat getrocknet. Nach Einengen wurde der Rückstand (519 mg eines braunen Öls) durch Säulenchromatographie an Kieselgel gereinigt (Laufmittel: Gradient Isohexan/4-32% Ethylacetat).
Ausbeute: 172 mg eines gelben Öls (20% d. Th.)
LC-MS (Methode 3): Rₜ = 1.62 min; MS (ESIpos): m/z = 440 [M+H]⁺.

### Beispiel 4A

### rac-5-{(5-Ethoxy-5-oxopentyl)[2-(2-hydroxyphenyl)ethyl]amino}-5,6,7,8-tetrahydronaphthalin-2-carbonsäureethylester

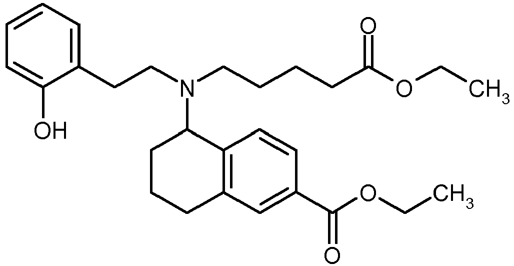

8.7 g (18.6 mmol) der Verbindung aus Beispiel 2A wurden in 90 ml einer 33%-igen Lösung von Bromwasserstoff in Eisessig über Nacht unter Rückfluss gerührt. Nach nochmaliger Zugabe von 90 ml der Bromwasserstoff-Lösung wurde eine weitere Nacht bei 135°C Badtemperatur gerührt. Danach wurde das Gemisch im Vakuum eingeengt, der Rückstand erneut mit 40 ml der Bromwasserstoff-Lösung in Eisessig versetzt und abermals über Nacht bei 110°C Badtemperatur gerührt. Anschließend wurde wiederum zur Trockene eingeengt. Der Rückstand, welcher zu 77% die Dicarbonsäure-Verbindung 5-{(4-Carboxybutyl)[2-(2-hydroxyphenyl)ethyl]amino}-5,6,7,8-tetrahydronaphthalin-2-carbonsäure [LC-MS (Methode 2): Rₜ = 0.64 min; MS (ESIpos): m/z = 412 [M+H]⁺] enthielt, wurde in 190 ml Ethanol angelöst und tropfenweise mit 1.65 ml Thionylchlorid versetzt. Das Gemisch wurde über Nacht bei 70°C gerührt und dann im Vakuum eingeengt. Der Rückstand (ca. 13 g) wurde in Ethylacetat aufgenommen und zweimal mit verdünnter wässriger Natriumcarbonat-Lösung gewaschen. Die organische Phase wurde über Natriumsulfat getrocknet und eingeengt. Das Rohprodukt (8.8 g) wurde mittels Flash-Chromatographie an Kieselgel mit Cyclohexan/Ethylacetat 5:1 als Laufmittel gereinigt.
Ausbeute: 5.3 g (56% d. Th.)
LC-MS (Methode 2): Rₜ = 0.86 min; MS (ESIpos): m/z = 468 [M+H]⁺
¹H-NMR (500 MHz, DMSO-d₆): δ [ppm] = 9.21 (s, 1H), 7.54-7.76 (m, 3H), 6.93-7.04 (m, 2H), 6.61-6.78 (m, 2H), 4.29 (q, 2H), 4.21-4.36 (m, 2H), 3.92-4.11 (m, 4H), 2.66-2.81 (m, 3H), 2.35-2.66 (m, 6H), 2.13-2.27 (m, 2H), 1.89-2.08 (m, 3H), 1.36-1.69 (m, 6H), 1.31 (t, 3H), 1.11-1.22 (m, 4H).

### Beispiel 5A

### rac-5-[(2-{2-[(4-tert.-Butylbenzyl)oxy]phenyl}ethyl)(5-methoxy-5-oxopentyl)amino]-5,6,7,8-tetrahydronaphthalin-2-carbonsäuremethylester-Ameisensäuresalz

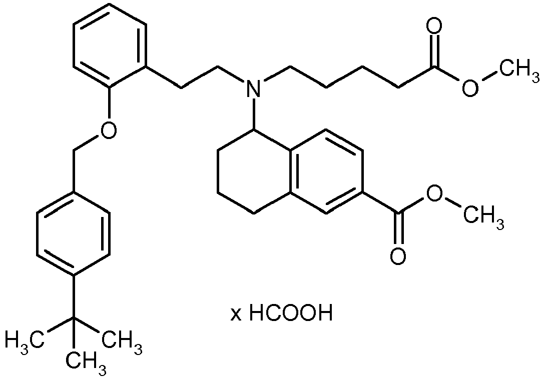

170 mg (0.387 mmol) der Verbindung aus Beispiel 3A, 104 mg (0.464 mmol) 4-*tert*.-Butylbenzylbromid und 403 mg (1.238 mmol) Cäsiumcarbonat wurden unter Argon in 3 ml DMF 2 h bei RT gerührt. Die Suspension wurde danach mit 30 ml Wasser versetzt und mit 5 N Ameisensäure auf pH 5 angesäuert. Es wurde mehrmals mit Ethylacetat extrahiert, und die vereinigten organischen Phasen wurden über Natriumsulfat getrocknet und im Vakuum eingeengt. Der Rückstand (340 mg eines gelben Öls) wurde über präparative HPLC (Methode 1) gereinigt.
Ausbeute: 133 mg (52% d. Th.)
LC-MS (Methode 2): Rₜ = 1.22 min; MS (ESIpos): m/z = 586 [M+H]⁺
¹H-NMR (400 MHz, DMSO-d₆): δ [ppm] = 8.15 (s, 1H), 7.60 (m, 3H), 7.33 (d, 2H), 7.23 (d, 2H), 7.04-7.18 (m, 2H), 6.97 (d, 1H), 6.83 (t, 1H), 4.93 (dd, 2H), 3.89-3.97 (m, 1H), 3.81 (s, 3H), 3.55 (s, 3H), 2.73-2.83 (m, 1H), 2.63-2.73 (m, 2H), 2.34-2.45 (m, 2H), 2.14-2.21 (m, 2H), 1.82-1.98 (m, 2H), 1.32-1.59 (m, 6H), 1.27 (s, 9H) [*weitere Signale vom DMSO-Peak überlagert*].

### Beispiel 6A und Beispiel 7A

### 5-[(2-{2-[(4-tert.-Butylbenzyl)oxy]phenyl}ethyl)(5-methoxy-5-oxopentyl)amino]-5,6,7,8-tetrahydronaphthalin-2-carbonsäuremethylester (Enantiomer 1 und 2)

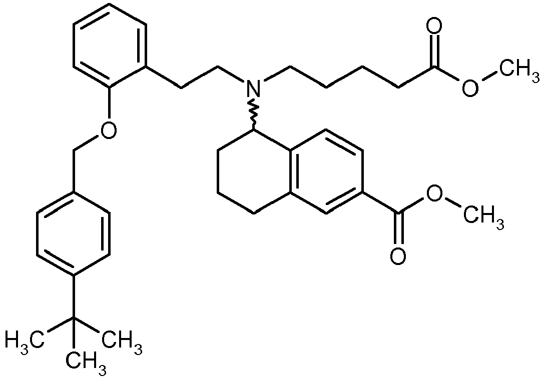

100 mg des racemischen 5-[(2-{2-[(4-*tert*.-Butylbenzyl)oxy]phenyl}ethyl)(5-methoxy-5-oxopentyl)-amino]-5,6,7,8-tetrahydronaphthalin-2-carbonsäuremethylesters aus Beispiel 5A wurden durch präparative HPLC an chiraler Phase in die Enantiomere aufgetrennt [Probenvorbereitung: die Substanz wurde in 3 ml Isopropanol und 7 ml Isohexan gelöst; Injektionsvolumen: je 1 ml; Säule: Daicel Chiralpak OD-H, 250 mm x 20 mm; Eluent: Isohexan/Isopropanol 50:50 (v/v); Fluss: 15 ml/min; Temperatur: 30°C; UV-Detektion: 210 nm]:

### Beispiel 6A (Enantiomere 1):

Ausbeute: 25.5 mg

HPLC (Methode 5): Rₜ = 4.34 min, >99% ee

¹H-NMR (400 MHz, DMSO-d₆): δ [ppm] = 7.60 (m, 3H), 7.33 (d, 2H), 7.24 (d, 2H), 7.04-7.18 (m, 2H), 6.97 (d, 1H), 6.83 (t, 1H), 4.92 (dd, 2H), 3.88-3.98 (m, 1H), 3.81 (s, 3H), 3.55 (s, 3H), 2.59-2.86 (m, 4H), 2.29-2.45 (m, 2H), 2.17 (t, 2H), 1.80-1.99 (m, 2H), 1.31-1.64 (m, 6H), 1.27 (s, 9H) [*weitere Signals vom DMSO-Peak überlagert*]*.*

### Beispiel 7A (Enantiomer 2):

Ausbeute: 22.6 mg

HPLC (Methode 5): Rₜ = 4.72 min, 90.5% ee

### Beispiel 8A

### rac-5-[(2-{2-[(4-tert.-Butylbenzyl)oxy]phenyl}ethyl)(5-ethoxy-5-oxopentyl)amino]-5,6,7,8-tetrahydronaphthalin-2-carbonsäureethylester

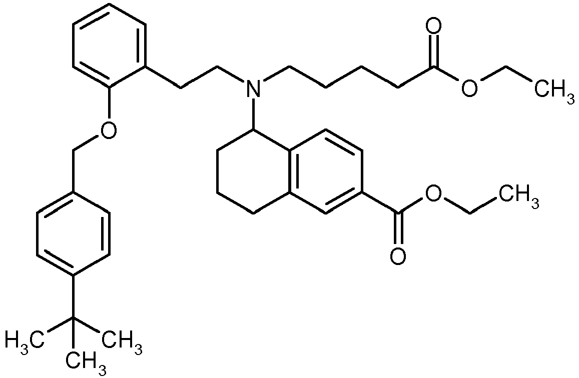

500 mg (1.07 mmol) der Verbindung aus Beispiel 4A wurden in 20 ml DMF gelöst, mit 245 µl (1.23 mmol) 4-*tert*.-Butylbenzylbromid sowie 1.12 g (3.4 mmol) Cäsiumcarbonat versetzt und über Nacht bei RT gerührt. Das Gemisch wurde danach mit Wasser versetzt. Nach Zugabe von 1.4 ml 5 N Ameisensäure wurde mehrfach mit Toluol extrahiert, und die vereinigten organischen Phasen wurden über Natriumsulfat getrocknet und eingeengt.
Ausbeute: 655 mg (92% Reinheit, 91% d. Th.)
LC-MS (Methode 2): Rₜ = 1.30 min; MS (ESIpos): m/z = 614 [M+H]⁺.

### Beispiel 9A und Beispiel 10A

### 5-[(2-{2-[(4-tert.-Butylbenzyl)oxy]phenyl}ethyl)(5-ethoxy-5-oxopentyl)amino]-5,6,7,8-tetrahydronaphthalin-2-carbonsäureethylester (Enantiomer 1 und 2)

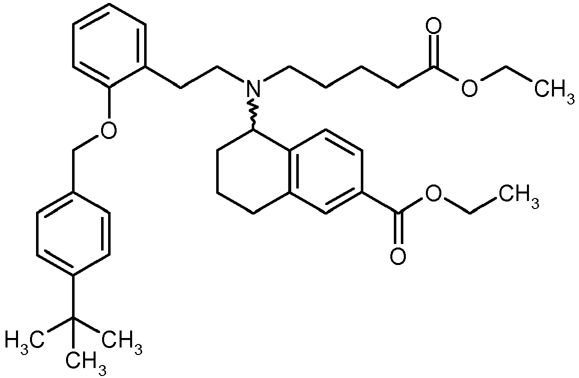

430 mg des racemischen 5-[(2-{2-[(4-*tert*.-Butylbenzyl)oxy]phenyl}ethyl)(5-ethoxy-5-oxopentyl)-amino]-5,6,7,8-tetrahydronaphthalin-2-carbonsäureethylesters aus Beispiel 8A wurden durch präparative HPLC an chiraler Phase in die Enantiomere aufgetrennt [Probenvorbereitung: die Substanz wurde in 20 ml Ethanol und 20 ml Isohexan gelöst; Injektionsvolumen: je 0.5 ml; Säule: Daicel Chiralpak OJ-H, 5 µm, 250 mm x 20 mm; Eluent: Isohexan/Ethanol/Methanol 95:2.5:2.5 (v/v/v); Fluss: 20 ml/min; Temperatur: 25°C; UV-Detektion: 230 nm]:

### Beispiel 9A (Enantiomer 1):

Ausbeute: 112 mg

HPLC (Methode 6): Rₜ = 5.65 min, >99.5% ee

LC-MS (Methode 2): Rₜ = 1.31 min; MS (ESIpos): m/z = 614 [M+H]⁺

¹H-NMR (500 MHz, DMSO-d₆): δ [ppm] = 7.60 (s, 3H), 7.34 (d, 2H), 7.24 (d, 2H), 7.15 (t, 1H), 7.10 (d, 1H), 6.98 (d, 1H), 6.82 (t, 1H), 4.94 (dd, 2H), 4.22-4.31 (m, 2H), 3.97-4.05 (m, 2H), 3.89-3.97 (m, 1H), 2.73-2.82 (m, 1H), 2.52-2.73 (m, 5H), 2.33-2.45 (m, 2H), 2.16 (t, 2H), 1.82-1.98 (m, 2H), 1.32-1.59 (m, 6H), 1.23-1.31 (m, 13H), 1.14 (t, 3H).

### Beispiel 10A (Enantiomer 2):

Ausbeute: 79 mg

HPLC (Methode 6): Rₜ = 7.095 min, 82.7% ee

LC-MS (Methode 2): Rₜ = 1.32 min; MS (ESIpos): m/z = 614 [M+H]⁺.

### Beispiel 11A

### rac-1-{[2-(2-Methoxyphenyl)ethyl]amino}indan-5-carbonsäureethylester

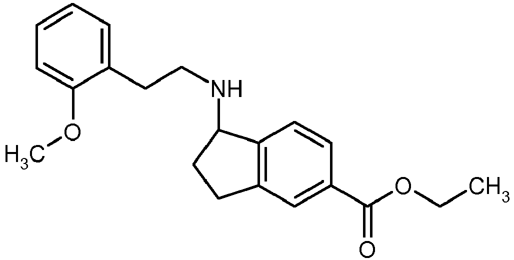

Die Titelverbindung wurde analog der Vorschrift für Beispiel 1A ausgehend von 2.0 g (9.73 mmol) 1-Oxoindan-5-carbonsäureethylester [R. Takeuchi und H. Yasue, J. Org. Chem. 1993, 58 (20), 5386-5392] hergestellt.
Ausbeute: 2.46 g eines bräunlichen Öls (Reinheit 87%)
LC-MS (Methode 3): Rₜ = 1.58 min; MS (ESIpos): m/z = 340 [M+H]⁺.

### Beispiel 12A

### rac-1-{(5-Ethoxy-5-oxopentyl)[2-(2-methoxyphenyl)ethyl]amino}indan-5-carbonsäureethylester

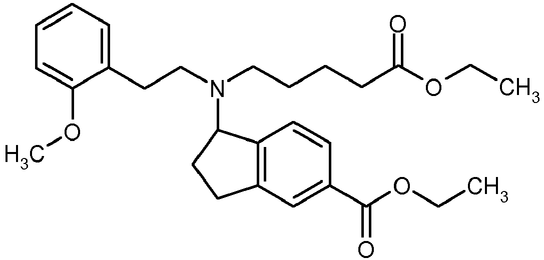

Die Titelverbindung wurde analog der Vorschrift für Beispiel 2A ausgehend von 2.2 g (6.48 mmol) der Verbindung aus Beispiel 11A hergestellt.
Ausbeute: 1.87 g (62% d. Th.) eines gelblichen Öls
LC-MS (Methode 2): Rₜ = 0.93 min; MS (ESIpos): m/z = 468 [M+H]⁺
¹H-NMR (400 MHz, DMSO-d₆): δ [ppm] = 7.69-7.79 (m, 2H), 7.11-7.19 (m, 2H), 7.07 (d, 1H), 6.89 (d, 1H), 6.82 (t, *J =* 7.34 Hz, 1H), 4.53 (t, 1H), 4.29 (q, 2H), 4.03 (q, 2H), 3.66 (s, 3H), 2.56-2.95 (m, 4H), 2.35-2.48 (m, 2H), 2.18-2.26 (m, 2H), 2.08-2.18 (m, 1H), 1.82-1.95 (m, 1H), 1.37-1.61 (m, 4H), 1.31 (t, 3H), 1.16 (t, 3H).

### Beispiel 13A

### rac-1-{(4-Carboxybutyl)[2-(2-hydroxyphenyl)ethyl]amino}indan-5-carbonsäure

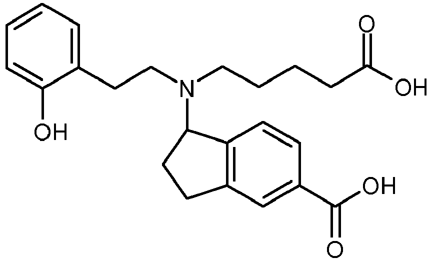

Analog der Vorschrift für Beispiel 3A wurden ausgehend von 1.8 g (3.85 mmol) der Verbindung aus Beispiel 12A 1.65 g des rohen Phenol-Diesters *rac*-1-{(5-Ethoxy-5-oxopentyl)[2-(2-hydroxyphenyl)ethyl]amino}indan-5-carbonsäureethylester [Gehalt 19%; LC-MS (Methode 2): Rₜ = 0.88 min; MS (ESIpos): m/z = 454 [M+H]⁺] erhalten. Dieses Rohprodukt wurde in 10 ml THF, 15 ml Methanol und 0.8 ml 5N Natronlauge 2 h lang unter Rückfluss erhitzt. Das Gemisch wurde danach auf ein Volumen von ca. 5 ml eingeengt und mit 5 N Ameisensäure auf pH 5-6 angesäuert. Dabei schied sich ein amorpher Niederschlag ab, der mittels Kieselgel-Chromatographie (Eluent Dichlormethan/4-34% Methanol) und anschließende präparative HPLC (Methode 1) gereinigt wurde.
Ausbeute: 64 mg (4% d. Th.) eines farblosen Feststoffs
LC-MS (Methode 2): Rₜ = 0.66 min; MS (ESIpos): m/z = 398 [M+H]⁺.

### Beispiel 14A

### rac-1-{(5-Ethoxy-5-oxopentyl)[2-(2-hydroxyphenyl)ethyl]amino}indan-5-carbonsäureethylester-Hydrochlorid

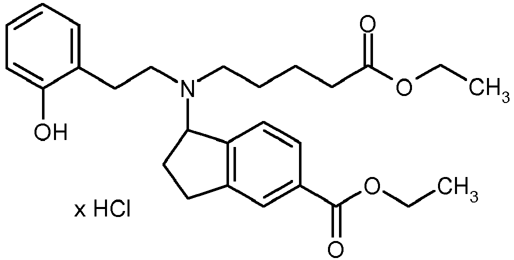

53 mg (0.133 mmol) der Verbindung aus Beispiel 13A wurden in 3 ml Ethanol gelöst, mit 19µl (0.27 mmol) Thionylchlorid versetzt und 4 h bei 75°C gerührt. Anschließend wurden nochmals 200 µl Thionylchlorid zugegeben und das Gemisch weiter über Nacht bei 75°C gerührt. Danach wurde bis zur Trockene eingeengt und der Rückstand zweimal mit Ethanol co-evaporiert.
Ausbeute: 60 mg (88% d. Th.) eines farblosen, amorphen Feststoffs
LC-MS (Methode 2): Rₜ = 0.88 min; MS (ESIpos): m/z = 454 [M+H]⁺
¹H-NMR (400 MHz, DMSO-d₆): δ [ppm] = 10.21-10.67 (breit, 1H), 7.83-8.06 (m, 3H), 6.96-7.20 (m, 2H), 6.65-6.90 (m, 2H), 5.20-5.42 (m, 1H), 4.33 (q, 2H), 3.96-4.13 (m, 2H), 2.85-3.21 (m, 7H), 2.68-2.81 (m, 1H), 2.34-2.44 (m, 2H), 2.23 (t, 1H), 1.38-1.97 (m, 4H), 1.33 (t, 3H), 1.07-1.22 (m, 3H) [*weitere Signals durch Wasser*- *und DMSO-Peak überlagert*].

Analog zur Vorschrift für Beispiel 5A wurden die folgenden Verbindungen hergestellt:

| **Beispiel** | **Edukte** | **Struktur** | **Ausbeute; analytische Daten** |
|---|---|---|---|
| **15A** | Bsp. 14A / 4-*tert*.-Butyl-benzylbromid | 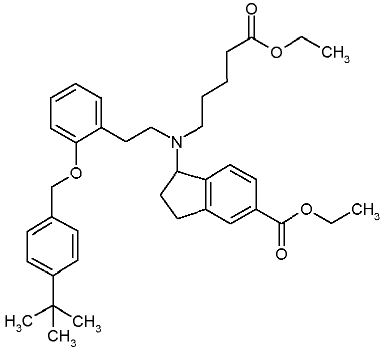 | 66% d. Th.; LC-MS (Methode 2): Rt = 1.20 min, m/z = 600 [M+H]⁺; ¹H-NMR (400 MHz, DMSO-d₆): δ [ppm] = 7.67-7.77 (m, 2H), 7.31-7.38 (m, 2H), 7.23-7.30 (m, 2H), 7.07-7.21 (m, 3H), 6.95-7.02 (m, 1H), 6.79-6.88 (m, 1H), 4.90-5.02 (m, 2H), 4.44-4.53 (m, 1H), 4.22-4.33 (m, 2H), 3.95-4.07 (m, 2H), 2.59-2.90 (m, 4H), 2.31-2.44 (m, 2H), 2.12-2.21 (m, 2H), 2.00-2.11 (m, 1H), 1.77-1.89 (m, 1H), 1.34-1.55 (m, 4H), 1.20-1.34 (m, 14H), 1.15 (s, 3H). |
| | | (*Racemat*) | |
| **16A** | Bsp. 4A / (3-Chlorpropyl)-pyrrolidin-2-on | 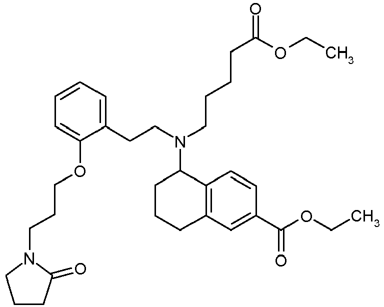 | 66% d. Th.; LC-MS (Methode 2): Rₜ = 0.98 min, m/z = 593 [M+H]⁺. |
| | | (*Racemat*) | |
| **17A** | Bsp. 16A¹⁾ | 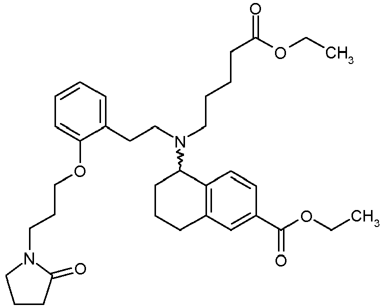 | 45% d. Th.; LC-MS (Methode 2): Rₜ = 1.01 1 min, m/z = 593 [M+H]⁺; HPLC (Methode 6): Rₜ = 4.89 min, 99.5% ee |
| | | (*Enantiomer 1*) | |
| **18A** | Bsp. 16A¹⁾ | 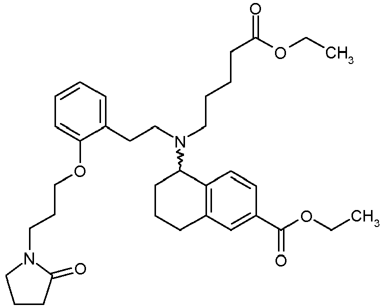 | 59% d. Th.; LC-MS (Methode 2): Rt = 1.01 min, m/z = 593 [M+H]⁺; HPLC (Methode 6): Rₜ = 7.16 min, 99.5% ee |
| | | (*Enantiomer 2*) | |
| **19A** | Bsp. 4A / 1-(Chlormethyl)-4-(2-phenylethyl)-benzol | 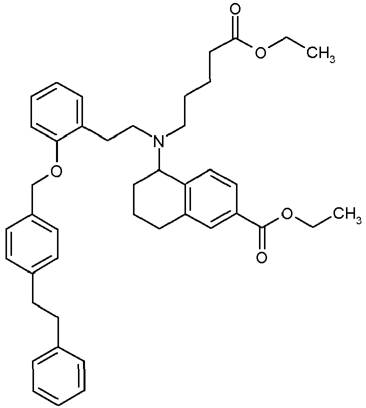 | 72% d. Th.; LC-MS (Methode 2): Rt = 1.34 min, m/z = 662 [M+H]⁺; ¹H-NMR (400 MHz, DMSO-d₆): δ [ppm] = 7.55-7.64 (m, 3H), 7.05-7.31 (m, 12H), 6.97 (d, 1H), 6.83 (t, 1H), 4.85-4.99 (m, 2H), 4.26 (q, 2H), 4.01 (q, 2H), 3.92 (br. s, 1H), 2.86 (s, 4H), 2.54-2.82 (m, 6H), 2.31-2.40 (m, 2H), 2.11-2.20 (m, 2H), 1.83-1.99 (m, 2H), 1.32-1.61 (m, 6H), 1.28 (t, 3H), 1.14 (t, 3H) [*Signale teilweise von DMSO-Peak überlagert*]*.* |
| | | (*Racemat*) | |
| **20A** | Bsp. 4A / 3-(3-Chlorpropyl)-1,3-oxazolidin-2-on | 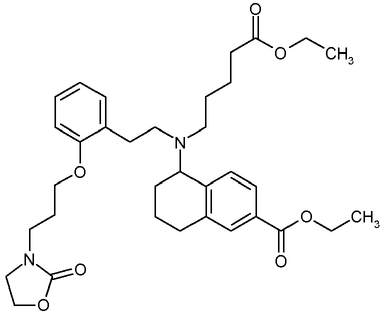 | 76% d. Th.; LC-MS (Methode 2): Rₜ = 0.97 min, m/z = 595 [M+H]⁺; ¹H-NMR (400 MHz, DMSO-d₆): δ [ppm] = 7.56-7.66 (m, 3H), 7.04-7.18 (m, 2H), 6.78-6.89 (m, 2H), 4.19-4.34 (m, 4H), 3.93-4.07 (m, 3H), 3.78-3.93 (m, 2H), 3.48 (t, 2H), 3.22 (t, 2H), 2.55-2.82 (m, 5H), 2.16-2.26 (m, 2H), 1.86-2.07 (m, 2H), 1.80 (quin, 2H), 1.36-1.68 (m, 6H), 1.31 (t, 3H), 1.15 (t, 3H) [*Signale teilweise von DMSO-Peak überlagert*]*.* |
| | | (*Racemat*) | |

| | | | |
|---|---|---|---|
| ¹) Methode Enantiomerentrennung: Probenvorbereitung: 71 mg des Racemats wurden in 8 ml Isopropanol gelöst und die Lösung mit 10 ml Isohexan versetzt; Injektionsvolumen: je 0.8 ml; Säule: Daicel Chiralpak OJ-H, 5 µm, 250 mm x 20 mm; Eluent: Isohexan/Ethanol/Methanol 50:25:25 (v/v/v); Fluss: 20 ml/min; Temperatur: 25°C; UV-Detektion: 230 nm. | | | |

### Ausführungsbeispiele:

### Beispiel 1

### rac-5-[(2-{2-[(4-tert.-Butylbenzyl)oxy]phenyl}ethyl)(4-carboxybutyl)amino]-5,6,7,8-tetrahydronaphthalin-2-carbonsäure

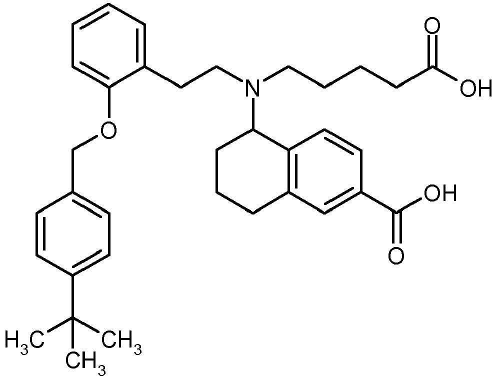

200 mg der Verbindung aus Beispiel 8A (92% Reinheit, 0.3 mmol) wurden in 4 ml THF und 2 ml Methanol gelöst und nach Zugabe von 345 µl (1.73 mmol) 5 N Natronlauge 1.5 h lang unter Rückfluss gerührt. Anschließend wurde mit Wasser verdünnt und dann das Lösungsmittel im Vakuum abgezogen. Man gab 650 µl 5 N Essigsäure hinzu, kühlte etwas ab, saugte den ausgefallenen farblosen Feststoff ab und trocknete diesen über Nacht im Hochvakuum.
Ausbeute: 172 mg (quant.)
LC-MS (Methode 2): Rₜ = 1.03 min; MS (ESIpos): m/z = 558 [M+H]⁺
¹H-NMR (400 MHz, DMSO-d₆): δ [ppm] = 7.53-7.64 (m, 3H), 7.35 (d, 2H), 7.24 (d, 2H), 7.15 (t, 1H), 7.09 (d, 1H), 6.98 (d, 1H), 6.83 (t, 1H), 4.93 (dd, 2H), 3.87-3.97 (m, 1H), 2.73-2.84 (m, 1H), 2.55-2.72 (m, 4H), 2.35-2.45 (m, 2H), 2.08-2.13 (m, 2H), 1.81-1.99 (m, 2H), 1.33-1.59 (m, 6H), 1.27 (s, 9H) [*Signale teilweise durch DMSO-Peak überlagert*].

### Beispiel 2

### 5-[(2-{2-[(4-tert.-Butylbenzyl)oxy]phenyl}ethyl)(4-carboxybutyl)amino]-5,6,7,8-tetrahydronaphthalin-2-carbonsäure (Enantiomer 1)

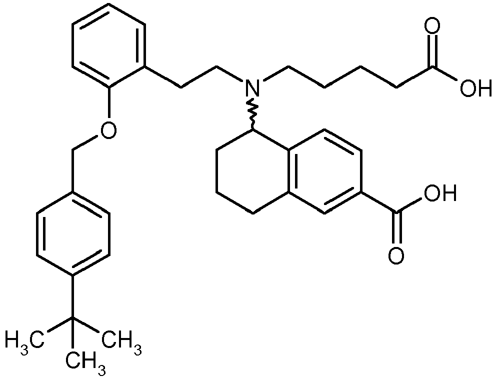

23 mg (0.039 mmol) der Verbindung aus Beispiel 6A wurden in 1 ml THF/Wasser (5:1) gelöst und mit 0.4 ml 1 N Natronlauge über Nacht bei RT gerührt. Danach wurden 0.2 ml Methanol zugesetzt und das Gemisch weitere 2 h bei RT gerührt. Man gab dann 0.08 ml 5 N Ameisensäure hinzu und engte bis zur Trockene ein. Der Rückstand wurde über präparative HPLC (Methode 1) gereinigt.
Ausbeute: 14 mg (62% d. Th.) eines farblosen Feststoffs
LC-MS (Methode 2): Rₜ = 1.02 min; MS (ESIpos): m/z = 558 [M+H]⁺
¹H-NMR (400 MHz, DMSO-d₆): δ [ppm] = 7.55-7.62 (m, 3H), 7.34 (d, 2H), 7.24 (d, 2H), 7.15 (t, 1H), 7.08 (d, 1H), 6.98 (d, 1H), 6.83 (t, 1H), 4.93 (dd, 2H), 3.88-3.97 (m, 1H), 2.73-2.83 (m, 1H), 2.58-2.73 (m, 4H), 2.39-2.46 (m, 2H), 2.09-2.15 (m, 2H), 1.82-1.99 (m, 2H), 1.32-1.58 (m, 6H), 1.27 (s, 9H) [*Signale teilweise durch DMSO-Peak überlagert*]*.*

### Beispiel 3

### 5-[(2-{2-[(4-tert.-Butylbenzyl)oxy]phenyl}ethyl)(4-carboxybutyl)amino]-5,6,7,8-tetrahydronaphthalin-2-carbonsäure (Enantiomer 2)

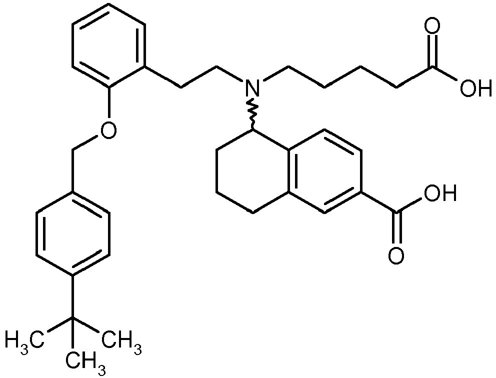

Die Titelverbindung wurde analog der Vorschrift für Beispiel 2 aus 21 mg (0.036 mmol) der Verbindung aus Beispiel 7A erhalten.
Ausbeute: 15.8 mg (79% d. Th.) eines farblosen Feststoffs
LC-MS (Methode 2): Rₜ = 1.02 min; MS (ESIpos): m/z = 558 [M+H]⁺.

Analog der Vorschriften für die Beispiele 1 und 2 wurden die folgenden Verbindungen hergestellt:

| **Beispiel** | **Struktur** | **Edukt** | **Ausbeute; analytische Daten** |
|---|---|---|---|
| **4** | 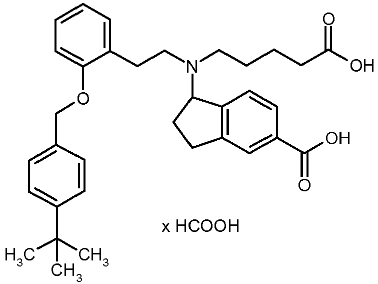 | 15A¹⁾⁴⁾ | 89% d. Th.; LC-MS (Methode 2): Rₜ = 0.97 min, m/z = 544 [M+H]⁺ |
| | (*Racemat*) | | |
| 5 | 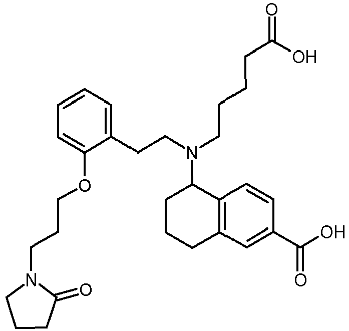 | 16A²⁾⁴⁾ | 93% d. Th.; LC-MS (Methode 2): Rt = 0.75 min, m/z = 537 [M+H]⁺ |
| | (*Racemat*) | | |
| 6 | 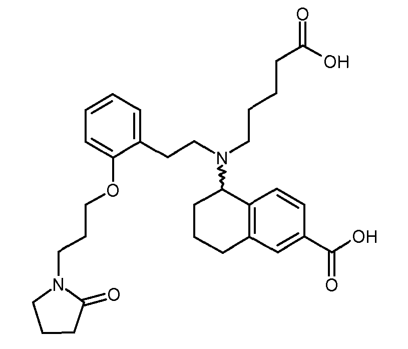 | 17A³⁾⁴⁾ | 20% d. Th.; LC-MS (Methode 2): Rₜ = 0.73 min, m/z = 537 [M+H]⁺ |
| | (*Enantiomer 1*) | | |
| 7 | 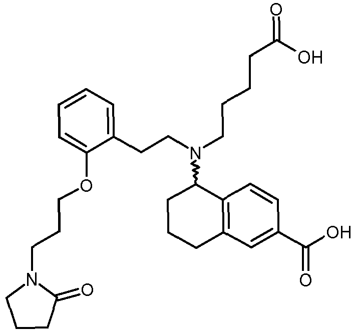 | 18A³⁾⁴⁾ | 26% d. Th.; LC-MS (Methode 2): Rₜ = 0.73 min, m/z = 537 [M+H]⁺ |
| | (*Enantiomer 2*) | | |
| **8** | 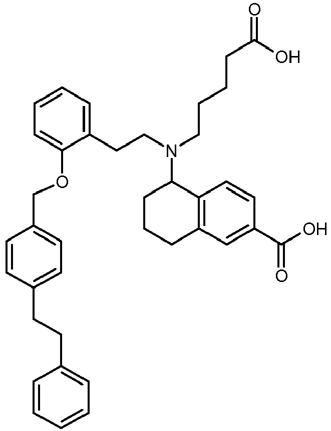 | 19A¹⁾⁴⁾ | 62% d. Th.; LC-MS (Methode 2): Rt = 1.05 min, m/z = 606 [M+H]^{+ 1}H-NMR (400 MHz, DMSO-d₆): δ [ppm] = 7.51-7.64 (m, 3H), 7.05-7.32 (m, 11H), 6.97 (d, 1H), 6.83 (t, 1H), 4.92 (dd, 2H), 3.91 (br. s, 1H), 2.82-2.92 (m, 4H), 2.55-2.82 (m, 6H), 2.43 |
| | (*Racemat*) | | (breit, 2H), 2.06-2.23 (m, 2H), 1.82-2.00 (m, 2H), 1.31-1.62 (m, 6H). |
| **9** | 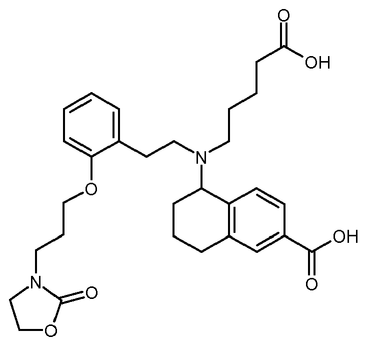 | 20A²⁾⁴⁾ | 72% d. Th.; LC-MS (Methode 2): Rt = 0.73 min, m/z = 539 [M+H]⁺ |
| | (*Racemat*) | | |

| | | | |
|---|---|---|---|
| ¹⁾ Die Ester-Hydrolyse erfolgte mit 5 N Natronlauge in THF/Methanol (1 h unter Rückfluss); ²⁾ Die Ester-Hydrolyse erfolgte mit 5 N Natronlauge in THF/Methanol (über Nacht bei RT); ³⁾ Die Ester-Hydrolyse erfolgte mit 5 N Natronlauge in DMSO (über Nacht bei RT); ⁴⁾ Die Reinigung des Rohprodukts erfolgte über präparative HPLC (Methode 1). | | | |

### Allgemeinen Vorschrift für die Herstellung weiterer Ausführungsbeispiele mittels Parallelsynthese:

Jeweils 1.2 Äquivalente (0.12 mmol) des betreffenden Alkylhalogenids wurden in einer Vertiefung einer 96er "deep well"-Mikrotiterplatte vorgelegt und mit einer Lösung von 47 mg (0.1 mmol) der Verbindung aus Beispiel 4A in 0.6 ml DMF versetzt. Zu diesem Gemisch wurden 44 mg (0.32 mmol) Kaliumcarbonat gegeben. Die Mikrotiterplatte wurde abgedeckt und über Nacht bei 80°C geschüttelt. Dann wurde abfiltriert, das Filtrat mit 0.6 ml 4 N Natronlauge versetzt, erneut abgedeckt und bei 60°C über Nacht geschüttelt. Danach wurde das Lösungsmittel abgedampft. Der Rückstand wurde in 0.6 ml DMSO aufgenommen und direkt über präparative LC-MS gereinigt (Methode 8 oder 9). Die produkthaltigen Fraktionen wurden mittels Zentrifugaltrockner im Vakuum eingeengt. Der Rückstand der einzelnen Fraktionen wurde in je 0.6 ml DMSO gelöst und vereinigt. Abschließend wurde im Zentrifugaltrockner das Lösungsmittel vollständig abgedampft.

Nach dieser Vorschrift wurden die folgenden Verbindungen erhalten:

| **Beispiel** | **Struktur** | **LC-MS (Methode 10)** |
|---|---|---|
| **10** | 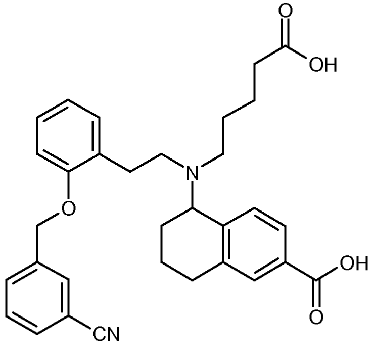 | Rₜ = 0.87 min, m/z = 527 [M+H]⁺ |
| | (*Racemat*) | |
| **11** | 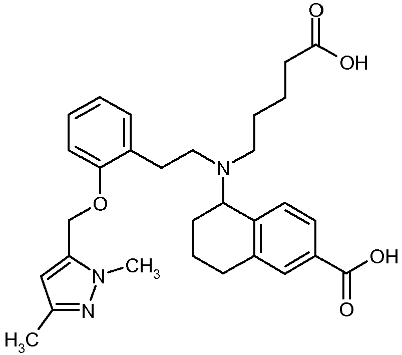 | Rₜ = 0.84 min, m/z = 520 [M+H]⁺ |
| | (*Racemat*) | |
| **12** | 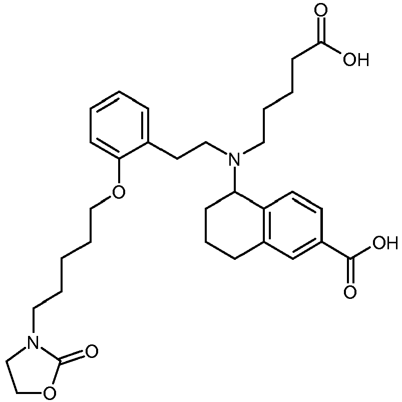 | Rₜ = 0.87 min, m/z = 567 [M+H]⁺ |
| | (*Racemat*) | |
| **13** | 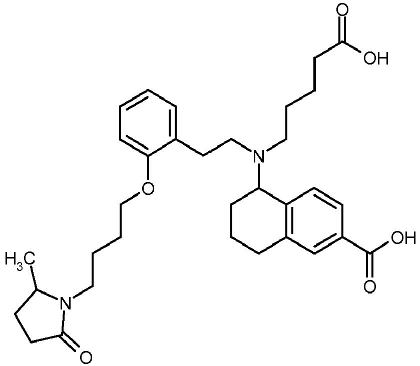 | Rₜ = 0.88 min, m/z = 565 [M+H]⁺ |
| | (*Racemat*) | |
| **14** | 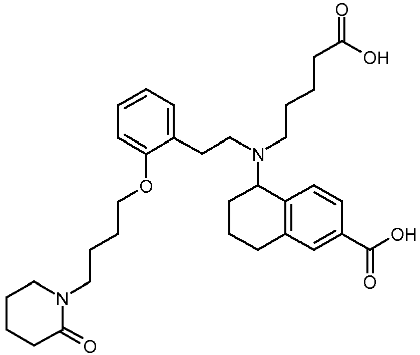 | Rₜ = 0.88 min, m/z = 565 [M+H]⁺ |
| | (*Racemat*) | |
| **15** | 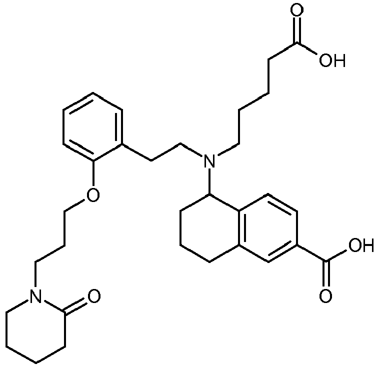 | Rₜ = 0.86 min, m/z = 551 [M+H]⁺ |
| | (*Racemat*) | |
| **16** | 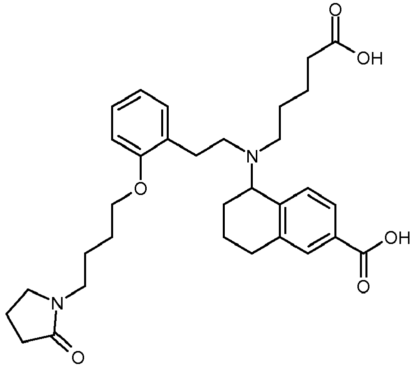 | Rₜ = 0.85 min, m/z = 551 [M+H]⁺ |
| | (*Racemat*) | |

### B. Bewertung der pharmakologischen Wirksamkeit

Die pharmakologische Wirkung der erfindungsgemäßen Verbindungen kann in folgenden Assays gezeigt werden:

### B-1. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Ergebnisse zu den erfindungsgemäßen Verbindungen sind in Tabelle 1 aufgeführt:

**Tabelle 1: sGC-aktivierende Wirkung in der CHO-Reporterzelle in vitro**

| **Beispiel Nr.** | **MEC [nM]** |
|---|---|
| 1 | 0.3 |
| 2 | 0.2 |
| 3 | 0.65 |
| 4 | 3.0 |
| 8 | 0.3 |
| 10 | 1000 |

| | |
|---|---|
| (MEC = minimale effektive Konzentration). | |

### B-2. Stimulation der sGC-Enzymaktivität

Lösliche Guanylatcyclase (sGC) setzt unter Stimulation GTP zu cGMP und Pyrophosphat (PPi) um. PPi wird mit Hilfe des nachfolgend beschriebenen Tests nachgewiesen. Das im Test entstehende Signal nimmt mit fortschreitender Umsetzung zu und dient als Maß für die sGC-Enzymaktivität unter der gegebenen Stimulation.

Zur Durchführung des Tests werden 29 µl Enzymlösung [0-10 nM lösliche Guanylatcyclase (hergestellt nach Hönicka et al., J. Mol. Med. 77, 14-23 (1999)) in 50 mM TEA, 2 mM MgCl₂, 0.1% BSA (Fraktion V), 0.005% Brij^{®}, pH 7.5] in eine Mikroplatte vorgelegt und 1 µl der zu testenden Substanz (als seriell verdünnte Lösung in DMSO) hinzugegeben. Der Ansatz wird 10 min bei Raumtemperatur inkubiert. Anschließend werden 20 µl Detektionsmix [1.2 nM Firefly-Luciferase (*Photinus pyralis*-Luciferase, Fa. Promega), 29 µM Dehydro-Luziferin (hergestellt nach Bitler & McElroy, Arch. Biochem. Biophys. 72, 358 (1957)), 122 µM Luziferin (Fa. Promega), 153 µM ATP (Fa. Sigma) und 0.4 mM DTT (Fa. Sigma) in 50 mM TEA, 2 mM MgCl₂, 0.1% BSA (Fraktion V), 0.005% Brij^{®}, pH 7.5] zugegeben. Die Enzymreaktion wird durch Zugabe von 20 µl Substratlösung [1.25 mM Guanosin-5'-triphosphat (Fa. Sigma) in 50 mM TEA, 2 mM MgCl₂, 0.1% BSA (Fraktion V), 0.005% Brij^{®}, pH 7.5] gestartet und kontinuierlich luminometrisch vermessen. Das Maß der Stimulation durch die zu testende Substanz kann relativ zum Signal der nicht stimulierten Reaktion bestimmt werden.

Durch Zugabe von 25 µM 1*H*-1,2,4-Oxadiazolo[4,3-a]chinoxalin-1-on (ODQ) zur Enzymlösung und anschließende 30-minütige Inkubation wird die Aktivierung der Häm-freien Guanylatcyclase untersucht und mit der Stimulation des nativen Enzyms verglichen.

Repräsentative Ergebnisse zu den erfindungsgemäßen Verbindungen sind in Tabelle 2 aufgeführt:

**Tabelle 2: Aktivierende Wirkung am sGC-Enzym in vitro**

| **Beispiel Nr.** | **MEC [nM]** | **EC₅₀ [nM]** |
|---|---|---|
| 1 | 0.34 | 3 |
| 2 | 0.11 | 2.5 |
| 3 | 5 | 43 |
| 4 | 0.21 | 4.5 |
| 8 | 0.32 | 7.2 |
| 10 | 270 | |
| 11 | 610 | |
| 13 | 55 | |
| 16 | 600 | |

| | | |
|---|---|---|
| (MEC = minimale effektive Konzentration; EC₅₀ = Konzentration bei 50% der maximalen Wirksamkeit). | | |

### B-3. Gefäßrelaxierende Wirkung in vitro

Kaninchen werden durch intravenöse Injektion von Thiopental-Natrium narkotisiert bzw. getötet (ca. 50 mg/kg) und entblutet. Die Arteria Saphena wird entnommen und in 3 mm breite Ringe geteilt. Die Ringe werden einzeln auf je einem triangelförmigen, am Ende offenen Häkchenpaar aus 0.3 mm starkem Spezialdraht (Remanium^{®}) montiert. Jeder Ring wird unter Vorspannung in 5 ml-Organbäder mit 37°C warmer, carbogenbegaster Krebs-Henseleit-Lösung folgender Zusammensetzung gebracht: NaCl 119 mM; KCl 4.8 mM; CaCl₂ x 2 H₂O 1 mM; MgSO₄ x 7 H₂O 1.4 mM; KH₂PO₄ 1.2 mM; NaHCO₃ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀-Wert). Das Standard-Applikationsvolumen beträgt 5 µl. Der DMSO-Anteil in der Badlösung entspricht 0.1%.

Repräsentative Ergebnisse zu den erfindungsgemäßen Verbindungen sind in Tabelle 3 aufgeführt:

**Tabelle 3: Gefäßrelaxierende Wirkung in vitro**

| **Beispiel Nr.** | **IC₅₀ [nM]** |
|---|---|
| 1 | 6 |
| 2 | 3.8 |
| 3 | 58 |
| 4 | 31 |

### B-4. Bronchodilatierende Wirkung in vitro und in vivo

### B-4.1 Bronchorelaxation in vitro

Es werden Bronchialringe (2-3 Segmente) von Ratte, Maus oder Meerschweinchen entnommen und einzeln auf je ein triangelförmiges, am Ende offenes Häkchenpaar aus 0.3 mm starkem Spezialdraht (Remanium^{®}) montiert. Jeder Ring wird unter Vorspannung in 5 ml-Organbäder mit 37°C warmer, carbogenbegaster Pufferlösung (z.B. Krebs-Henseleit-Lösung) gebracht. Die Bronchialringe werden mit Methacholin (1 µM) vorkontrahiert, um dann die Bronchorelaxation durch Zugabe von steigenden Konzentrationen (10⁻⁹ bis 10⁻⁶ M) der jeweiligen Testsubstanz zu untersuchen. Die Ergebnisse werden als prozentuale Relaxation in Bezug auf die Vorkonstriktion durch Methacholin ausgewertet.

### B-4.2 Tierexperimentelle Prüfung zur Untersuchung der Wirkung auf die Bronchokonstriktion im Asthmamodell

Alle Tiere (Ratten, Mäuse) werden vor Beginn des Provokationstests intragastral mit der Schlundsonde oder inhalativ behandelt. Dabei erhalten die Tiere der Behandlungsgruppen die Prüfsubstanz, die Kontrolltiere erhalten entsprechend eine Vehikel-Lösung. Nach Ablauf der Wartephase werden die Tiere narkotisiert und intubiert. Nach Legen eines Oesophagus-Katheters und Erreichen eines Steady state der Atmung wird zunächst die Lungenfunktion vor Provokation gemessen. Messparameter sind u.a. die Lungenresistance (RL) und die dynamische Compliance (Cdyn) sowie Atemzugvolumen (VT) und Atemfrequenz (f). Die Datenspeicherung und die statistische Auswertung erfolgen mit speziell für diese Lungenfunktionstests entwickelten Rechnerprogrammen (Notocord HEM).

Dann folgt eine definierte inhalative Exposition der Versuchstiere gegenüber einem Methacholin (MCh)-Aerosol (Modell einer unspezifisch induzierten asthmatischen Bronchokonstriktion). Während und 3 min nach der Exposition wird die Aufzeichnung der Lungenfunktionsparameter fortgesetzt. MCh-Konzentration und -Dosis in der Inhalationsluft werden mit einem entwickelten Feedback-Dosiskontrollsystem gesteuert und überwacht (mittels Messung der Aerosolkonzentration und des Minutenvolumens). Bei Erreichen der Zieldosis wird der Test beendet. Anhand des Resistanceanstiegs wird im Vergleich mit der scheinbehandelten Positivkontrolle die inhibitorische Wirkung der Testsubstanzen ermittelt.

### Studie im allergischen Asthmamodell:

Alle Tiere bis auf die Negativkontrolle werden mit dem Allergen Ovalbumin und Adjuvans (Alum) systemisch sensibilisiert. Die Negativkontrollgruppe erhält stattdessen physiologische KochsalzLösung (NaCl). Alle Gruppen werden dann mit Ovalbumin provoziert. In der Studie werden 6 Behandlungsgruppen - 2 Prüfsubstanzen in je 3 Dosisgruppen - eingesetzt, dazu wird eine mit Dexamethason i.p. behandelte Referenzgruppe, eine scheinbehandelte und -provozierte Negativkontrollgruppe und eine scheinbehandelte und mit Ovalbumin provozierte Positivkontrollgruppe eingesetzt. Sensibilisierungs-, Behandlungs- und Challengeprotokoll: An Tag 0, 14 und 21 werden alle Tiere mit Ovalbumin und Adjuvans i.p. sensibilisiert, die Negativkontrolle wird mit NaCl behandelt. An Tag 28 und 29 werden die Tiere mit einer intratrachealen Gabe einer Ovalbumin-Lösung provoziert. Die Prüfsubstanzen werden 1 h vor jeder intratrachealen Allergenprovokation intragastral oder inhalativ verabreicht. Eine Referenzgruppe wird 18 h und 1 h vor jeder intratrachealen Allergenprovokation mit Dexamethason i.p. behandelt. Die Positiv- und die Negativkontrollgruppe werden entsprechend mit dem Vehikel behandelt.

### Atemwegshyperreaktivität und inflammatorische Antwort:

Zunächst werden die Tiere auf Hyperreaktivität der Atemwege gegenüber unspezifischen Stimuli untersucht. Dazu wird ca. 24 h nach Ovalbumin-Challenge ein Hyperreaktivitätstest in Form einer stufenweise ansteigenden inhalativen Methacholin-Provokation durchgeführt.

Die Tiere werden narkotisiert, orotracheal intubiert und die Lungenfunktion vor Provokation bodyplethysmographisch gemessen (incl. Parametern wie Atemzugvolumen, Atemfrequenz, dynamischer Compliance und Lungenresistance). Nach Abschluss der Messungen wird für jedes Tier die Dosiswirkungskurve erstellt und die Hyperreaktivität der Positivkontrolle gegenüber der Negativkontrolle bzw. deren Hemmung in den Behandlungsgruppen ausgewertet.

Danach werden die Tiere schmerzlos getötet, Blutproben entnommen und die Lungen lavagiert (BAL). Aus der Lavageflüssigkeit wird die Gesamtzellzahl und das Differentialzellbild incl. Anzahl der Eosinophilen in der BAL bestimmt. Die restlichen Mengen an BAL-Flüssigkeit werden zunächst eingefroren. Es können dann gegebenenfalls nachträglich noch weitere Parameter (z.B. Zytokine) bestimmt werden. Das Lungengewebe wird für eine optionale histopathologische Untersuchung asserviert.

### B-5. Isoliert perfundiertes Herz nach Langendorff

Männliche Wistar-Ratten (Stamm HsdCpb:WU) mit einem Körpergewicht von 200-250 g werden mit Narcoren^{®} (100 mg/kg) narkotisiert. Nach Eröffnen des Thorax wird das Herz frei präpariert, ausgeschnitten und durch Kanulieren der Aorta an eine Langendorff-Apparatur angeschlossen. Das Herz wird retrograd mit einer Krebs-Henseleit-Pufferlösung (begast mit 95% O₂ und 5% CO₂, pH 7.4, 35°C; Zusammensetzung in mmol/l: NaCl 118; KCl 3; NaHCO₃ 22; KH₂PO₄ 1.2; MgSO₄ 1.2; CaCl₂ 1.8; Glucose 10; Na-Pyruvat 2) mit 9 ml/min flusskonstant perfundiert. Für die Erfassung der Kontraktionskraft des Herzens wird durch eine Öffnung im linken Herzohr ein an einem PE-Schlauch befestigter und mit Wasser gefüllter Ballon aus dünner Kunststofffolie in den linken Ventrikel eingeführt. Der Ballon wird mit einem Druckaufnehmer verbunden. Der enddiastolische Druck wird auf 5-10 mmHg über das Ballonvolumen eingestellt. Der Perfusionsdruck wird mit Hilfe eines zweiten Druckaufnehmers detektiert. Die Daten werden über einen Brückenverstärker an einen Computer gesendet und registriert.

Nach 40 min Äquilibrierungszeit wird die jeweilige Testsubstanz in einer Endkonzentration von 10⁻⁷ mol/l der Perfusionslösung für 20 min hinzugefügt, was als Zeichen der koronaren Dilatation zu einer Reduktion des Perfusionsdruckes führt. Danach werden die Herzen für weitere 120 min ohne Testsubstanz perfundiert (Auswaschphase). Für die Bestimmung der Reversibilität der Perfusionsdrucksenkung (Wash out-Score) wird der Wert des Perfusionsdruckes nach 60 min der Auswaschphase auf die maximale Perfusionsdrucksenkung durch die Testsubstanz bezogen und prozentuell dargestellt. Der so erhaltene Wash out-Score wird als Maß für die Verweildauer der Testsubstanz am Wirkort gewertet.

### B-6. Hämodynamik im narkotisierten Ferkel

Es werden 2-6 kg schwere, gesunde Göttinger Minipigs^{®} Ellegaard (Ellegaard, Dänemark) beiderlei Geschlechts verwendet. Die Tiere werden sediert durch i.m.-Gabe von ca. 25 mg/kg Ketamin und ca. 10 mg/kg Azaperon. Die Narkose-Einleitung erfolgt durch i.v.-Gabe von ca. 2 mg/kg Ketamin und ca. 0.3 mg/kg Midazolam. Die Narkose-Erhaltung erfolgt durch i.v.-Gabe von ca. 7.5-30 mg/kg/h Ketamin und ca. 1-4 mg/kg/h Midazolam (Infusionsrate 1-4 ml/kg/h) sowie ca. 150 µg/kg/h Pancuroniumbromid (z.B. Pancuronium-Actavis). Nach der Intubation werden die Tiere über die Beatmungsmaschine mit konstantem Atemvolumen beatmet (10-12 ml/kg, 35 Atemzüge/min; Avea^{®}, Viasys Healthcare, USA, oder Engström Carestation, GE Healthcare, Freiburg, Deutschland), so dass eine endtidale CO₂-Konzentration von etwa 5% erreicht wird. Die Beatmung erfolgt mit Raumluft, angereichert mit ca. 40% Sauerstoff (Normoxie). Zur Messung der hämodynamischen Parameter, wie z.B. pulmonal-arterieller Druck (PAP), Blutdruck (BP) und Herzfrequenz (HR), werden Katheter in die A. carotis zur Messung des Blutdrucks und ein Swan-Ganz^{®}-Katheter über die V. jugularis in die Pulmonalarterie eingeschwemmt. Die hämodynamischen Signale werden mittels Druckaufnehmern (Combitransducer, B. Braun, Melsungen, Deutschland) / Verstärkern und Ponemah^{®} als Datenerfassungssoftware aufgezeichnet und ausgewertet.

Nach Beendigung der Instrumentierung der Tiere wird zur Erhöhung des pulmonal-arteriellen Drucks eine Dauerinfusion mit einem Thromboxan A₂-Analogon gestartet. Es werden ca. 0.3-0.75 µg/kg/min 9,11-Dideoxy-9α,11α-epoxymethanoprostaglandin F_{2α} (U-44069; Sigma, Kat.-Nr. D0400, oder Cayman Chemical Company, Kat.-Nr. 16440), gelöst in physiologischer KochsalzLösung, infundiert, um einen Anstieg des mittleren pulmonal-arteriellen Drucks auf Werte über 25 mmHg zu erreichen. 30 Minuten nach Start der Infusion stellt sich ein Plateau ein, und das Experiment wird gestartet.

Die Testsubstanzen werden als i.v.-Infusion oder per Inhalation verabreicht. Zur Herstellung der Inhalationslösung wird folgendermaßen vorgegangen: Für ein 4 kg schweres Tier wird zur Herstellung der Stammlösung (300 µg/kg) 1.2 mg der Testverbindung eingewogen und in 3 ml Gesamtvolumen (1% DMSO, 99% 0.2%-ige Zitronensäurelösung, 1 N Natronlauge zur Adjustierung des pH auf 8) gelöst. Die Lösung wird dann mit 0.2%-iger Zitronensäure, die zuvor mit Natronlauge auf pH 8 eingestellt wurde, bis zur eingesetzten Konzentration verdünnt. Pro Versuch werden pro 4 kg-Tier 3 ml der Testverbindungslösung mittels des Aeroneb^{®} Pro-Verneblersystems im Inhalationsschenkel des Beatmungskreislaufs vernebelt. Die mittlere Vernebelungszeit beträgt ca. 7 min nach Start der Vernebelung.

### B-7. Inhalative Gabe von sGC-Aktivatoren in PAH-Tiermodellen

Die Experimente werden in narkotisierten Göttinger Minischweinen, narkotisierten Ratten oder wachen, telemetrisch instrumentierten Hunden durchgeführt. Akute pulmonale Hypertonie wird z.B. durch Infusion eines Thromboxan A₂-Analogons, durch akute oder mehrwöchige Hypoxiebehandlung und/oder durch Monocrotalin-Gabe induziert. Die Vernebelung der Testsubstanzen erfolgt unter Verwendung des Nebutec^{®}- oder Aeroneb^{®} Pro-Verneblersystems, über Pulver- und/ oder Lösungsapplikatoren zur experimentellen intratrachealen Applikation (Liquid MicroSprayer^{®}, Dry Powder Insufflator™, MicroSprayer^{®}, Penn-Century Inc., Wyndmoor, PA, USA) oder nach Feststoffvernebelung, die in den Inspirationsschenkel der Beatmung geschaltet werden. Die Substanzen werden in Abhängigkeit von der molekularen Struktur als Feststoffe oder Lösungen eingesetzt. Die hämodynamischen Signale werden mittels Druckaufnehmern (Combitransducer, B. Braun, Melsungen, Deutschland) / Verstärkern und Ponemah^{®} oder CardioMems^{®} als Datenerfassungssoftware aufgezeichnet und ausgewertet. Nach Langzeitexperimenten (z.B. Monocrotalin-Ratte) kann auch eine histologische Auswertung erfolgen.

### B-8. Radiotelemetrische Messung von Blutdruck und Herzfrequenz an wachen Ratten

Für die im Folgenden beschriebenen Messungen an wachen Ratten wird ein im Handel erhältliches Telemetriesystem der Firma Data Sciences International DSI, USA, eingesetzt. Das System besteht aus 3 Hauptkomponenten: (*1*) Implantierbare Sender (Physiotel^{®} Telemetrietransmitter), (2) Empfänger (Physiotel^{®} Receiver), die über einen Multiplexer (DSI Data Exchange Matrix) mit einem (*3*) Datenakquisitionscomputer verbunden sind. Die Telemetrieanlage ermöglicht eine kontinuierliche Erfassung von Blutdruck, Herzfrequenz und Körperbewegung an wachen Tieren in ihrem gewohnten Lebensraum.

Die Untersuchungen werden an ausgewachsenen weiblichen Wistar-Ratten mit einem Körpergewicht von > 200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

### Senderimplantation:

Die eingesetzten Telemetriesender (TA11 PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal^{®}, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum (Oxytetracyclin^{®} 10%, 60 mg/kg s.c., 0.06 ml/100 g Körpergewicht, Beta-Pharma GmbH, Deutschland) sowie ein Analgetikum (Rimadyl^{®}, 4 mg/kg s.c., Pfizer, Deutschland) verabreicht.

### Substanzen und Lösungen:

Wenn nicht anders beschrieben, werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

### Versuchsablauf:

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (*1*) systolischer Blutdruck (SBP), (2) diastolischer Blutdruck (DBP), (3) arterieller Mitteldruck (MAP), (4) Herzfrequenz (HR) und (5) Aktivität (ACT).

Die Messwerterfassung wird rechnergesteuert in 5 Minuten-Abständen wiederholt. Die als Absolutwert erhobenen Quelldaten werden im Diagramm mit dem aktuell gemessenen Barometerdruck (Ambient Pressure Reference Monitor, APR-1) korrigiert und in Einzeldaten abgelegt. Weitere technische Details sind in der umfangreichen Dokumentation der Herstellerfirma (DSI) aufgeführt.

Wenn nicht anders beschrieben, erfolgt die Verabreichung der Prüfsubstanzen am Versuchstag um 9:00 Uhr. Im Anschluss an die Applikation werden die oben beschriebenen Parameter über 24 Stunden gemessen.

### Auswertung:

Nach Versuchsende werden die erhobenen Einzeldaten mit der Analysis-Software (Dataquest™ A.R.T. 4.1 Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur:

K. Witte, K. Hu, J. Swiatek, C. Müssig, G. Ertl und B. Lemmer, Experimental heart failure in rats: effects on cardiovascular circadian rhythms and on myocardial β-adrenergic signaling, Cardiovasc. Res. 47 (2): 350-358 (2000).

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 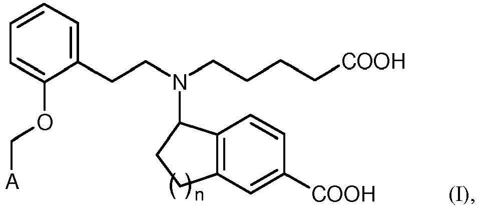 in welcher
n für die Zahl 1 oder 2 steht,
und
A für eine Gruppe der Formel 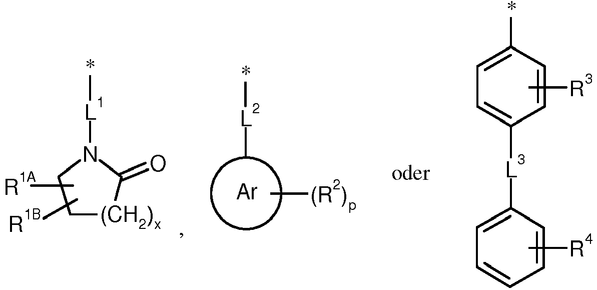
steht, worin
* die jeweilige Verknüpfungsstelle mit dem Rest des Moleküls kennzeichnet,
L¹ geradkettiges (C₁-C₅)-Alkandiyl bedeutet,
x die Zahl 1, 2 oder 3 bedeutet, wobei eine dieser CH₂-Gruppen gegen -O-ausgetauscht sein kann,
R^{1A} und R^{1B} unabhängig voneinander Wasserstoff oder Methyl bedeuten,
L² eine Bindung oder geradkettiges (C₁-C₅)-Alkandiyl bedeutet,
Ar Phenyl oder 5- oder 6-gliedriges Heteroaryl mit bis zu drei Heteroatomen aus der Reihe N, O und/oder S bedeutet,
R² einen Substituenten ausgewählt aus der Reihe Fluor, Chlor, Brom, Cyano, (C₁-C₄)-Alkyl, Trifluormethyl, (C₁-C₄)-Alkoxy und Trifluormethoxy bedeutet,
p die Zahl 0, 1 oder 2 bedeutet, wobei im Falle, dass der Substituent R² zweifach auftritt, seine jeweiligen Bedeutungen gleich oder verschieden sein können,
L³ eine Bindung, -O-, -CH₂-, -CH₂-CH₂- oder -CH=CH- bedeutet,
und
R³ und R⁴ unabhängig voneinander Wasserstoff oder einen Substituenten ausgewählt aus der Reihe Fluor, Chlor, Brom, Cyano, (C₁-C₄)-Alkyl, Trifluormethyl, (C₁-C₄)-Alkoxy und Trifluormethoxy bedeuten,
sowie ihre Salze, Solvate und Solvate der Salze.

2. Verbindung der Formel (I) nach Anspruch 1, in welcher
n für die Zahl 1 oder 2 steht,
und
A für eine Gruppe der Formel 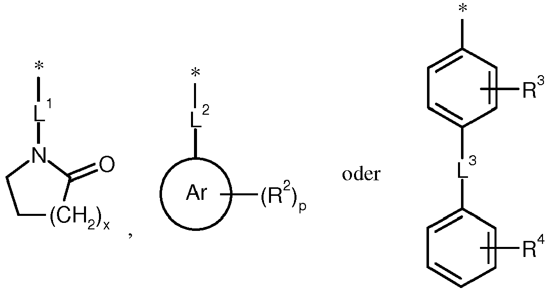
steht, worin
* die jeweilige Verknüpfungsstelle mit dem Rest des Moleküls kennzeichnet,
L¹ geradkettiges (C₂-C₄)-Alkandiyl bedeutet,
x die Zahl 1 oder 2 bedeutet, wobei eine dieser CH₂-Gruppen gegen -O- ausgetauscht sein kann,
L² eine Bindung oder geradkettiges (C₁-C₄)-Alkandiyl bedeutet,
Ar Phenyl bedeutet,
R² einen Substituenten ausgewählt aus der Reihe Fluor, Chlor, Cyano, (C₁-C₄)-Alkyl und Trifluormethyl bedeutet,
p die Zahl 0 oder 1 bedeutet,
L³ eine Bindung oder -CH₂-CH₂- bedeutet,
und
R³ und R⁴ unabhängig voneinander Wasserstoff oder einen Substituenten ausgewählt aus der Reihe Fluor, Chlor, Cyano, (C₁-C₄)-Alkyl und Trifluormethyl bedeuten,
sowie ihre Salze, Solvate und Solvate der Salze.

3. Verbindung der Formel (I) nach Anspruch 1 oder 2, in welcher
n für die Zahl 1 oder 2 steht,
und
A für eine Gruppe der Formel 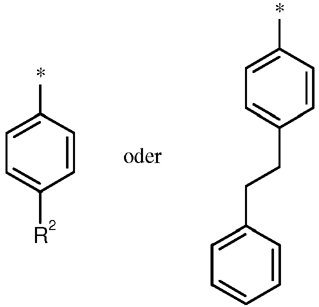
steht, worin
* die jeweilige Verknüpfungsstelle mit dem Rest des Moleküls kennzeichnet
und
R² Methyl, Ethyl, Isopropyl oder *tert*.-Butyl bedeutet,
sowie ihre Salze, Solvate und Solvate der Salze.

4. Verfahren zur Herstellung einer Verbindung der Formel (I), wie in den Ansprüchen 1 bis 3 definiert, **dadurch gekennzeichnet, dass** man eine Verbindung der Formel (II) 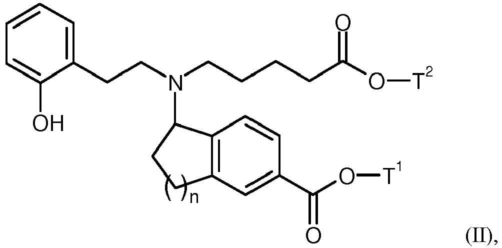 in welcher n die in den Ansprüchen 1 bis 3 angegebenen Bedeutungen hat
und
T¹ und T² gleich oder verschieden sind und für (C₁-C₄)-Alkyl stehen,
in Gegenwart einer Base mit einer Verbindung der Formel (III) 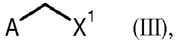 in welcher A die in den Ansprüchen 1 bis 3 angegebenen Bedeutungen hat
und
X¹ für eine Abgangsgruppe wie Chlor, Brom, Iod, Mesylat, Triflat oder Tosylat steht,
zu einer Verbindung der Formel (IV) 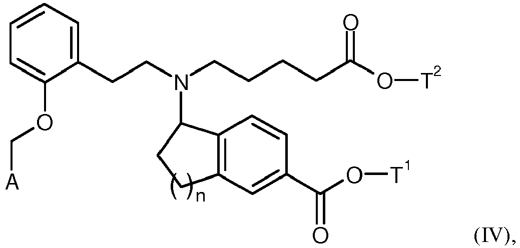 in welcher n, A, T¹ und T² jeweils die oben angegebenen Bedeutungen haben,
umsetzt und diese dann durch Hydrolyse der Ester-Gruppierungen -C(O)OT¹ und -C(O)OT² in die entsprechende Dicarbonsäure der Formel (I) überführt
und die so erhaltenen Verbindungen der Formel (I) gegebenenfalls in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

5. Verbindung, wie in einem der Ansprüche 1 bis 3 definiert, zur Verwendung bei der Behandlung und/oder Prävention von Krankheiten.

6. Verbindung, wie in Anspruch 5 definiert, zur Verwendung in einem Verfahren zur Behandlung und/oder Prävention von Herzinsuffizienz, Angina pectoris, Hypertonie, pulmonaler Hypertonie, thromboembolischen Erkrankungen, Ischämien, Gefäßerkrankungen, Mikrozirkulationsstörungen, Niereninsuffizienz, fibrotischen Erkrankungen und Arteriosklerose.

7. Verwendung einer Verbindung, wie in einem der Ansprüche 1 bis 3 definiert, zur Herstellung eines Arzneimittels zur Behandlung und/oder Prävention von Herzinsuffizienz, Angina pectoris, Hypertonie, pulmonaler Hypertonie, thromboembolischen Erkrankungen, Ischämien, Gefäßerkrankungen, Mikrozirkulationsstörungen, Niereninsuffizienz, fibrotischen Erkrankungen und Arteriosklerose.

8. Arzneimittel enthaltend eine Verbindung, wie in einem der Ansprüche 1 bis 3 definiert, in Kombination mit einem oder mehreren inerten, nicht-toxischen, pharmazeutisch geeigneten Hilfsstoffen.

9. Arzneimittel enthaltend eine Verbindung, wie in Anspruch 8 definiert, in Kombination mit einem oder mehreren weiteren Wirkstoffen ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln sowie den Fettstoffwechsel verändernden Mitteln.

10. Arzneimittel nach Anspruch 8 oder 9 zur Verwendung bei der Behandlung und/oder Prävention von Herzinsuffizienz, Angina pectoris, Hypertonie, pulmonaler Hypertonie, thromboembolischen Erkrankungen, Ischämien, Gefäßerkrankungen, Mikrozirkulationsstörungen, Niereninsuffizienz, fibrotischen Erkrankungen und Arteriosklerose.

## Claims

1. Compound of the formula (I) 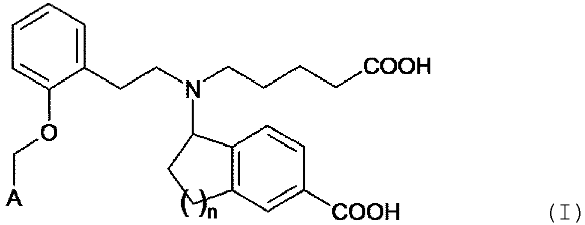 in which
n represents the number 1 or 2
and
A represents a group of the formula 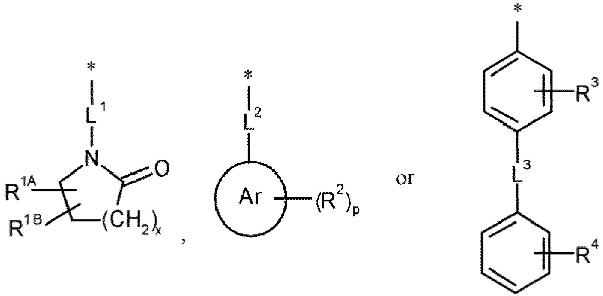
in which
* denotes the respective point of attachment to the remainder of the molecule,
L¹ represents straight-chain (C₁-C₅)-alkanediyl,
x represents the number 1, 2 or 3, where one of these CH₂ groups may be replaced by -O-,
R^{1A} and R^{1B} independently of one another represent hydrogen or methyl,
L² represents a bond or straight-chain (C₁-C₅)-alkanediyl,
Ar represents phenyl or 5- or 6-membered heteroaryl having up to three heteroatoms from the group consisting of N, O and S,
R² represents a substituent selected from the group consisting of fluorine, chlorine, bromine, cyano, (C₁-C₄)-alkyl, trifluoromethyl, (C₁-C₄)-alkoxy and trifluoromethoxy,
p represents the number 0, 1 or 2, where in the case where the substituent R² occurs twice, its respective meanings can be identical or different,
L³ represents a bond, -O-, -CH₂-, -CH₂-CH₂-or -CH=CH-
and
R³ and R⁴ independently of one another represent hydrogen or a substituent selected from the group consisting of fluorine, chlorine, bromine, cyano, (C₁-C₄)-alkyl, trifluoromethyl, (C₁-C₄)-alkoxy and trifluoromethoxy,
and their salts, solvates and solvates of the salts.

2. Compound of the formula (I) according to Claim 1 in which
n represents the number 1 or 2
and
A represents a group of the formula 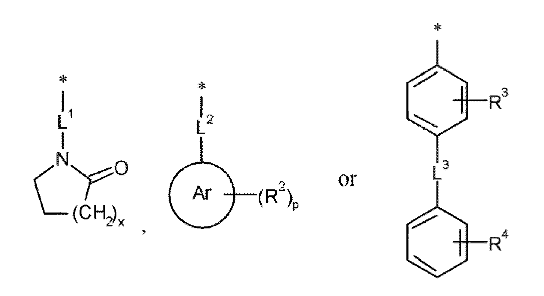
in which
* denotes the respective point of attachment to the remainder of the molecule,
L¹ represents straight-chain (C₂-C₄)-alkanediyl,
x represents the number 1 or 2, where one of these CH₂ groups may be replaced by-O-,
L² represents a bond or straight-chain (C₁-C₄)-alkanediyl,
Ar represents phenyl,
R² represents a substituent selected from the group consisting of fluorine, chlorine, cyano, (C₁-C₄)-alkyl and trifluoromethyl,
p represents the number 0 or 1,
L³ represents a bond or -CH₂-CH₂-
and
R³ and R⁴ independently of one another represent hydrogen or a substituent selected from the group consisting of fluorine, chlorine, cyano, (C₁-C₄)-alkyl and trifluoromethyl,
and their salts, solvates and solvates of the salts.

3. Compound of the formula (I) according to Claim 1 or 2 in which
n represents the number 1 or 2
and
A represents a group of the formula 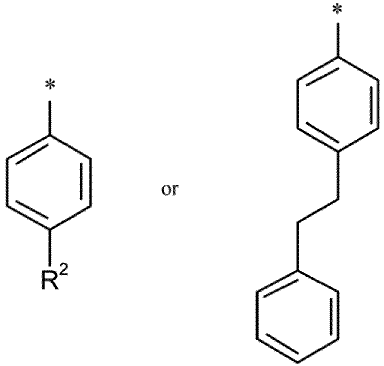
in which
* denotes the respective point of attachment to the remainder of the molecule
and
R² represents methyl, ethyl, isopropyl or *tert*-butyl,
and their salts, solvates and solvates of the salts.

4. Process for preparing a compound of the formula (I) as defined in any of Claims 1 to 3, **characterized in that** a compound of the formula (II) 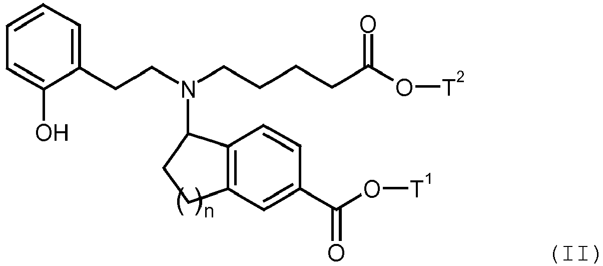 in which n has the meanings given in any of Claims 1 to 3
and
T¹ and T² are identical or different and represent (C₁-C₄)-alkyl
is reacted in the presence of a base with a compound of the formula (III) 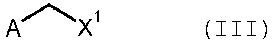 in which A has the meanings given in any of Claims 1 to 3
and
X¹ represents a leaving group, such as chlorine, bromine, iodine, mesylate, triflate or tosylate,
to give a compound of the formula (IV) 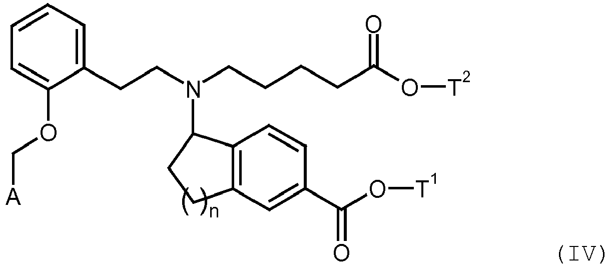 in which n, A, T¹ and T² each have the meanings given above,
and this is then converted by hydrolysis of the ester groupings -C(O)OT¹ and -C(O)OT² into the corresponding dicarboxylic acid of the formula (I)
and the resulting compounds of the formula (I) are optionally separated into the enantiomers and/or diastereomers thereof and/or optionally reacted with the appropriate (i) solvents and/or (ii) bases or acids to give the solvates, salts and/or solvates of the salts thereof.

5. Compound as defined in any of Claims 1 to 3 for use in the treatment and/or prevention of diseases.

6. Compound as defined in Claim 5 for use in a method for the treatment and/or prevention of heart failure, angina pectoris, hypertension, pulmonary hypertension, thromboembolic disorders, ischaemias, vascular disorders, impaired microcirculation, renal insufficiency, fibrotic disorders and arteriosclerosis.

7. Use of a compound as defined in any of Claims 1 to 3 for production of a medicament for treatment and/or prevention of heart failure, angina pectoris, hypertension, pulmonary hypertension, thromboembolic disorders, ischaemias, vascular disorders, impaired microcirculation, renal insufficiency, fibrotic disorders and arteriosclerosis.

8. Medicament comprising a compound as defined in any of Claims 1 to 3 in combination with one or more inert, nontoxic, pharmaceutically suitable excipients.

9. Medicament comprising a compound as defined in Claim 8 in combination with one or more further active compounds selected from the group consisting of organic nitrates, NO donors, cGMP-PDE inhibitors, stimulators of guanylate cyclase, antithrombotic agents, hypotensive agents and lipid metabolism modifiers.

10. Medicament according to Claim 8 or 9 for use in the treatment and/or prevention of heart failure, angina pectoris, hypertension, pulmonary hypertension, thromboembolic disorders, ischaemias, vascular disorders, impaired microcirculation, renal insufficiency, fibrotic disorders and arteriosclerosis.

## Revendications

1. Composé de formule (I) 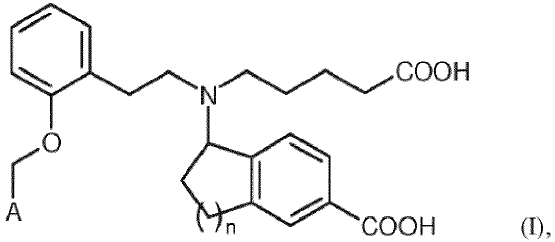 dans laquelle
n représente le nombre 1 ou 2,
et
A représente un groupe de formule 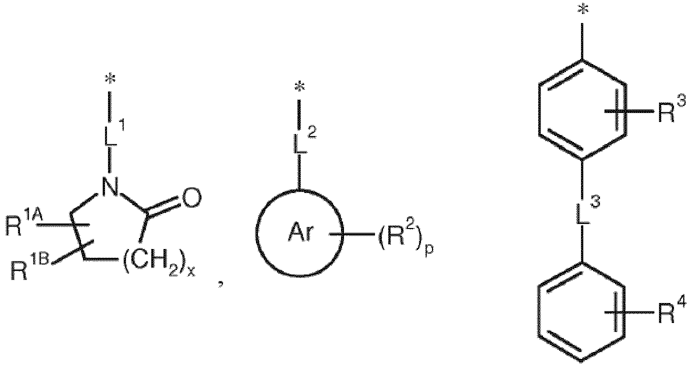
ou
dans lesquelles
* caractérise l'emplacement de liaison respectif avec le reste de la molécule,
L¹ signifie alcanediyle en (C₁-C₅) linéaire,
x signifie le nombre 1, 2 ou 3, un de ces groupes CH₂ pouvant être remplacé par -O-,
R^{1A} et R^{1B} signifient indépendamment l'un de l'autre hydrogène ou méthyle,
L² signifie une liaison ou alcanediyle en (C₁-C₅) linéaire,
Ar signifie phényle ou hétéroaryle à 5 ou 6 éléments avec jusqu'à trois hétéroatomes de la série constituée par N, O et/ou S,
R² signifie un substituant choisi dans la série constituée par fluor, chlore, brome, cyano, alkyle en (C₁-C₄), trifluorométhyle, alcoxy en (C₁-C₄) et trifluorométhoxy,
p signifie le nombre 0, 1 ou 2,
lorsque le substituant R² apparaît à deux reprises, ses significations respectives pouvant être identiques ou différentes,
L³ signifie une liaison, "O-, -CH₂-, -CH₂-CH₂- ou-CH=CH-,
et
R³ et R⁴ signifient indépendamment l'un de l'autre l'hydrogène ou un substituant choisi dans la série constituée par fluor, chlore, brome, cyano, alkyle en (C₁-C₄), trifluorométhyle, alcoxy en (C₁-C₄) et trifluorométhoxy,
ainsi que ses sels, solvates et solvates des sels.

2. Composé de formule (I) selon la revendication 1, dans lequel
n représente le nombre 1 ou 2,
et
A représente un groupe de formule 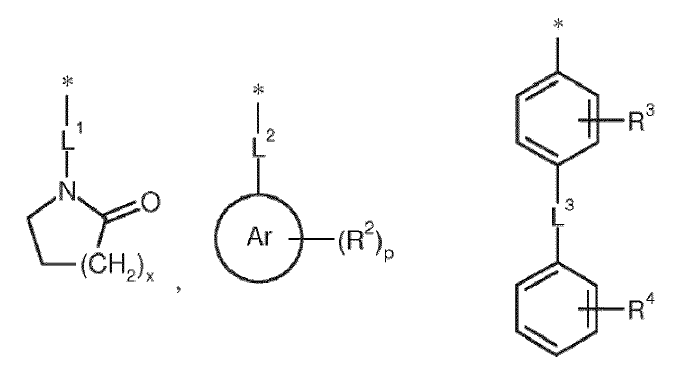
ou
dans lesquelles
* caractérise l'emplacement de liaison respectif avec le reste de la molécule,
L¹ signifie alcanediyle en (C₂-C₄) linéaire,
x signifie le nombre 1 ou 2, un de ces groupes CH₂ pouvant être remplacé par -O-,
L² signifie une liaison ou alcanediyle en (C₁-C₄) linéaire,
Ar signifie phényle,
R² signifie un substituant choisi dans la série constituée par fluor, chlore, cyano, alkyle en (C₁-C₄) et trifluorométhyle,
p signifie le nombre 0 ou 1,
L³ signifie une liaison ou -CH₂-CH₂-,
et
R³ et R⁴ signifient indépendamment l'un de l'autre l'hydrogène ou un substituant choisi dans la série constituée par fluor, chlore, cyano, alkyle en (C₁-C₄) et trifluorométhyle,
ainsi que ses sels, solvates et solvates des sels.

3. Composé de formule (I) selon la revendication 1 ou 2, dans lequel
n représente le nombre 1 ou 2,
et
A représente un groupe de formule 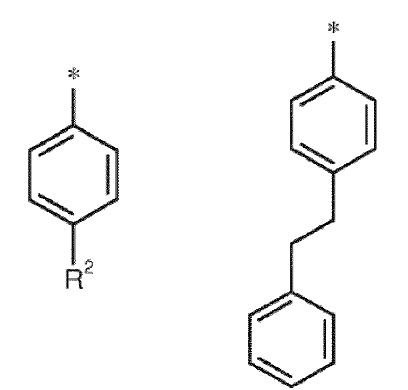
ou
dans lesquelles
* caractérise l'emplacement de liaison respectif avec le reste de la molécule,
et
R² signifie méthyle, éthyle, isopropyle ou *tert.-*butyle,
ainsi que ses sels, solvates et solvates des sels.

4. Procédé de fabrication d'un composé de formule (I), tel que défini dans les revendications 1 à 3, **caractérisé en ce qu'**un composé de formule (II) 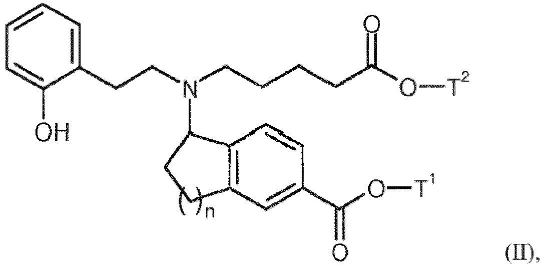 dans laquelle n a les significations indiquées dans les revendications 1 à 3,
et
T¹ et T² sont identiques ou différents, et représentent alkyle en (C₁-C₄),
est mis en réaction en présence d'une base avec un composé de formule (III) 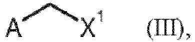 dans laquelle A les significations indiquées dans les revendications 1 à 3,
et
X¹ représente un groupe partant, tel que chlore, brome, iode, mésylate, triflate ou tosylate,
pour former un composé de formule (IV) 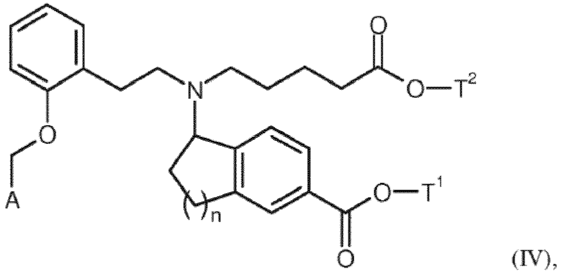 dans laquelle n, A, T¹ et T² ont chacun les significations indiquées précédemment,
puis celui-ci est transformé par hydrolyse des groupes ester -C(O)OT¹ et -C(O)OT² en l'acide dicarboxylique correspondant de formule (I),
et les composés de formule (I) ainsi obtenus sont éventuellement séparés en leurs énantiomères et/ou diastéréomères et/ou éventuellement transformés avec (i) les solvants et/ou (ii) les bases ou les acides appropriés en leurs solvates, sels et/ou solvates des sels.

5. Composé, tel que défini dans l'une quelconque des revendications 1 à 3, destiné à une utilisation pour le traitement et/ou la prévention de maladies.

6. Composé, tel que défini dans la revendication 5, destiné à une utilisation dans un procédé de traitement et/ou de prévention de l'insuffisance cardiaque, de l'angine de poitrine, de l'hypertension, de l'hypertension pulmonaire, des maladies thromboemboliques, des ischémies, des maladies vasculaires, des troubles de la microcirculation, de l'insuffisance rénale, des maladies fibrotiques et de l'artériosclérose.

7. Utilisation d'un composé, tel que défini dans l'une quelconque des revendications 1 à 3, pour la fabrication d'un médicament pour le traitement et/ou la prévention de l'insuffisance cardiaque, de l'angine de poitrine, de l'hypertension, de l'hypertension pulmonaire, des maladies thromboemboliques, des ischémies, des maladies vasculaires, des troubles de la microcirculation, de l'insuffisance rénale, des maladies fibrotiques et de l'artériosclérose.

8. Médicament contenant un composé, tel que défini dans l'une quelconque des revendications 1 à 3, en combinaison avec un ou plusieurs adjuvants inertes, non toxiques, pharmaceutiquement appropriés.

9. Médicament contenant un composé, tel que défini dans la revendication 8, en combinaison avec un ou plusieurs autres agents actifs choisis dans le groupe constitué par les nitrates organiques, les donneurs de NO, les inhibiteurs de cGMP-PDE, les stimulateurs de guanylate cyclase, les agents antithrombotiques, les agents abaissant la tension artérielle, ainsi que les agents modifiant le métabolisme des lipides.

10. Médicament selon la revendication 8 ou 9, destiné à une utilisation pour le traitement et/ou la prévention de l'insuffisance cardiaque, de l'angine de poitrine, de l'hypertension, de l'hypertension pulmonaire, des maladies thromboemboliques, des ischémies, des maladies vasculaires, des troubles de la microcirculation, de l'insuffisance rénale, des maladies fibrotiques et de l'artériosclérose.
